# EUROPEAN PATENT APPLICATION

(11) **EP 1 980 243 A1**
(43) Date of publication of application: **15.10.2008**
(21) Application number: 07713965.7
(22) Date of filing: 02.02.2007
(51) Int. Cl.: A61K 9/127, A61K 31/282, A61K 31/337, A61K 31/513, A61K 31/537, A61K 31/573, A61K 31/7068, A61K 33/36, A61K 45/00, A61K 47/18, A61K 47/24, A61K 47/26, A61K 47/28, A61K 47/36, A61K 47/42, A61P 35/00

(54) **LIPOSOME PREPARATION**

(30) Priority: 03.02.2006 JP 2006027231
(71) Applicant: Takeda Pharmaceutical Company Limited, Osaka 541-0045 (JP); National Institute of Advanced Industrial Science and Technology, Tokyo 100-8921 (JP)
(72) Inventor: YAMAZAKI, Noboru c/o National Institute of Advanced Industrial, Ibraki, 305-8565 (JP); YOSHIDA, Sei c/o TAKEDA PHARMACEUTICAL CO. LTD., Ibaraki, 300-4293 (JP); YAMAOKA, Masuo c/o TAKEDA PHARMACEUTICAL CO. LTD., Ibaraki, 300-4293 (JP); HORI, Akira c/o TAKEDA PHARMACEUTICAL CO. LTD., Ibaraki, 300-4293 (JP); KAKIMOTO, Shigeya, Ibaraki 300-4293 (JP); FURUYA, Shuichi c/o TAKEDA PHARMACEUTICAL CO. LTD., Ibaraki, 300-4293 (JP)
(74) Representative: Keller, Günter
(86) International application number: PCT/JP2007/052303
(87) International publication number: WO 2007/089043

(57) **Abstract**

The present invention provides cancer treatment preparations of excellent targetability. The sugar chain-modified liposomes of the present invention, which contain an aromatase inhibitor, anti-androgenic agent, lyase inhibitor, GnRH agonist, GnRH antagonist, anti-angiogenic agent, tyrosine kinase inhibitor, serine-threonine kinase inhibitor, antibody having an anticancer activity, ansamitocin, capecitabine, celmoleukin, docetaxel hydrate, gemcitabine hydrochloride, oxaliplatin, prednisolone, tegafur-uracil mixtures, zinostatin stimalamer or arsenic trioxide may be used as cancer treatment preparations having an excellent targetability.

## Description

### TECHNICAL FIELD

The present invention relates to liposomes containing a drug having an anticancer activity, and to liposomal preparations containing such liposomes.

### BACKGROUND OF THE INVENTION

The U.S. National Nanotechnology Initiative (NNI) cites, as one specific goal it aims to achieve, "a drug or gene delivery system (DDS) for targeting cancer cells and target tissues." Similarly, the Japanese Council for Science and Technology Policy includes, as a priority among its strategies for advancement in nanotechnology and materials: "Nanobiology which utilizes and controls very small systems, materials and biomechanisms for medical treatment," and mentions, as one five-year goal for research and development in this area: "the establishment of basic seeds on biofunctional materials and pinpoint therapy for prolonging health and life." At the same time, with the cancer morbidity and mortality rising from to year to year in an aging society, there exists a desire for the development of targeted drug delivery systems (DDS) as novel therapeutic materials. The significance of targeted DDS nanomaterials without side effects has attracted attention in other diseases as well, and it is predicted that the scale of this market will in the near future exceed 10 trillion yen. Moreover, along with treatment, these materials also show promise in diagnostic applications.

The therapeutic effects of a drug product appears as a result of the delivery of the drug to a specific target site and its action at that site. On the other hand, the side effects of a drug product are due to the action of the drug at unnecessary sites. Accordingly, the development of drug delivery systems is desired also for the safe and effective use of drugs. Of these, the targeted DDS delivers, in particular, the concept of a "required amount" of a drug "to a required site in the body" and "for exactly a required length of time." Liposomes, which are microparticulate carriers, have drawn attention as a leading material for this purpose. To impart such particles with a targeting function, passive targeting methods such as varying the type of lipid, constituent ratio, particle diameter and surface charge for the liposome are being attempted. However, the methods used, to date, fall short of what is needed; further improvements are awaited.

Active targeting methods are also being tried in order to enable high-function targeting. This is an ideal targeting method which is also referred to as a "missile drug." Because it has yet to be perfected in Japan and elsewhere, future developments are eagerly awaited. This method enables active targeting by coupling ligands to the liposome membrane surface and inducing specific recognition by receptors present on cell membrane surfaces of the target tissue. Receptor ligands present on the surface of the cell membrane targeted by this active targeting method may include, for example, antigens, antibodies, peptides, glycolipids and glycoproteins. Ongoing research is showing that, of these, the sugar chains on glycolipids and glycoproteins play important roles as information molecules in various kinds of intercellular communication, such as the development and morphogenesis of living tissue, cell growth and division, the mechanisms of biological defense and fertilization, and the mechanisms of cancer formation and metastasis.

As a result of advances in research on receptors present on the surface of the cell membrane in various target tissues, including various types of lectins (sugar chain recognition proteins), such as C-type lectins (e.g., selectin, DC-SIGN, DC-SGNR, collectin, mannose-binding proteins), I-type lectins (e.g., Siglec), P-type lectins (e.g., mannose-6-phosphate receptors), R-type lectins, L-type lectins, M-type lectins and galectin, sugar chains of various molecular structures are attracting attention as novel DDS ligands (Adv. Drug Delivery Rev. 43, 225-244 (2000); Trends in Glycoscience and Glycotechnology 13, 319-329 (2001)).

The following liposomes with sugar chains attached to the outer membrane surface have been reported in the literature: a sugar chain-modified liposome in which the sugar chains are attached to the liposome membrane via a linker protein, the sugar chains are selected from Lewis X trisaccharide, sialyl Lewis X tetrasaccharide, 3'-sialyllactosamine trisaccharide and 6'-sialyllactosamine trisaccharide, and tris(hydroxymethyl)aminomethane is optionally attached to the liposome membrane and/or a linker protein to hydrophilize (i.e., to make it hydrophilic) (Japanese Patent Application Laid-open No. 2003-226638; U.S. Published Patent Application No. 2003/0143267); and a targeted liposome wherein sialyl Lewis X saccharide, or sugar chains that can similarly be reacted with E-selectin, P-selectin or the like are attached under control of the sugar chain type and density, which liposome has a targetability to a site of inflammatory disease, is selectively taken up at such a site, releases an encapsulated drug at the site, and is capable of treating the site (PCT International Patent Application No. 2005/011633). In addition, a sugar chain-modified liposome which encapsulates the anticancer drug doxorubicin, has attached thereon sugar chains with specific binding activities to various lectins (sugar chain recognition proteins) present on the cell surfaces in various types of tissues, and is capable of differentiating cells and tissue in vivo and efficiently delivering a drug or gene have also been reported (WO 2005/011632).

Another key area of research on new types of DDS materials is the development of DDS materials which can be administered orally, enabling administration to be carried out in the most convenient and inexpensive way. For example, because peptidic and proteinaceous drug products are generally water-soluble, have a high molecular weight, and have a low permeability through the small intestinal mucosa of the digestive tract, due to enzymatic degradation and the like, even when administered orally, substantially no intestinal tract absorption takes place. Hence, research on ligand-coupled liposomes as a DDS material for delivering these high-molecular-weight drug products, genes and the like from the intestinal tract to the blood is starting to attract attention (J. Controlled Release 65, 19-29 (2000)).

The following intestinal absorption-controlled liposomes which use sugar chains as ligands have been reported in the literature: intestinal absorption-controlled liposomes which are modified with sugar chains selected from among lactose disaccharide, 2'-fucosyllactose trisaccharide, difucosyllactose tetrasaccharide and 3-fucosyllactose trisaccharide, wherein the sugar chain may be attached to the liposome through a linker protein (U.S. Published Patent Application No. 2003/0143267; Japanese Patent Application Laid-open No. 2003-226647); and sugar chain-modified liposomes wherein the sugar chains are attached to the liposome membrane, and the intestinal absorption is controlled by appropriate selection of the type of sugar chains used and the amount of sugar chains attached to the liposome (WO 2005/011632).

### DISCLOSURE OF THE INVENTION

With regard to drugs having anticancer activities, such as aromatase inhibitors, anti-androgenic agents, lyase inhibitors, GnRH agonists, GnRH antagonists, anti-angiogenic agents, tyrosine kinase inhibitors, serine-threonine kinase inhibitors, antibodies having anticancer activities, ansamitocin, capecitabine, celmoleukin, docetaxel hydrate, gemcitabine hydrochloride, oxaliplatin, prednisolone, tegafur-uracil mixtures, zinostatin stimalamer and arsenic trioxide, there exists a desire for both drug preparations which have an excellent targetability and drug preparations which have intestinal absorption controllability.

The inventors have conducted intensive investigations to resolve the above challenges. As a result, they have discovered that sugar chain-modified liposomes containing the above drugs exhibit excellent targetability to tissues or organs such as the blood, liver, spleen, lungs, brain, small intestine, heart, thymus, kidneys, pancreas, muscle, large intestine, bones, bone marrow, eyes, ovaries, placenta, prostate, pituitary gland, mammary glands, blood vessels, skin, gall bladder, bile ducts, bladder, adipose tissue, cancer tissue, inflamed tissue and lymph nodes, and also exhibit excellent intestinal absorption controllability. Based on these findings, the inventors conducted further investigations, ultimately arriving at the present invention.

Accordingly, the invention provides:
[1] A sugar chain-modified liposome comprising a sugar chain attached to a liposome membrane, the liposome containing at least one selected from among aromatase inhibitors, anti-androgenic agents, lyase inhibitors, GnRH agonists, GnRH antagonists, anti-angiogenic agents, tyrosine kinase inhibitors, serine-threonine kinase inhibitors, antibodies having anticancer activities, ansamitocin, capecitabine, celmoleukin, docetaxel hydrate, gemcitabine hydrochloride, oxaliplatin, prednisolone, tegafur-uracil mixtures, zinostatin stimalamer and arsenic trioxide; [1a] A sugar chain-modified liposome comprising a sugar chain attached to a liposome membrane, which liposome contains docetaxel hydrate;
[2] The sugar chain-modified liposome according to [1] above, wherein constituent lipids in the liposome include phosphatidylcholines (molar ratio, 0 to 70%); phosphatidylethanolamines (molar ratio, 0 to 30%); one or more lipid selected from the group consisting of phosphatidic acids, long-chain alkyl phosphates and dicetyl phosphates (molar ratio, 0 to 30%); one or more lipid selected from the group consisting of gangliosides, glycolipids, phosphatidylglycerols and sphingomyelins (molar ratio, 0 to 40%); and cholesterols (molar ratio, 0 to 70%);
[3] The sugar chain-modified liposome according to [2] above, wherein at least one type of lipid selected from the group consisting of gangliosides, glycolipids, phosphatidylglycerols, sphingomyelins and cholesterols collect on a surface of the liposome to form a raft.
[4] The sugar chain-modified liposome according to any one of [1] to [3] above, wherein the sugar chains are attached under control of the type and density thereof;
[5] The sugar chain-modified liposome according to any one of [1] to [4] above, wherein the liposome has a particle size of from 30 to 500 nm;
[6] The sugar chain-modified liposome according to [5] above, wherein the liposome has a particle size of from 50 to 350 nm;
[7] The sugar chain-modified liposome according to any one of [1] to [6] above, wherein the liposome has a zeta potential of from -50 to 10 mV;
[8] The sugar chain-modified liposome according to [7] above, wherein the liposome has a zeta potential of from -40 to 0 mV;
[9] The sugar chain-modified liposome according to [8] above, wherein the liposome has a zeta potential of from -30 to -10 mV;
[10] The sugar chain-modified liposome according to any one of [1] to [9] above, wherein the sugar chain is attached to the liposome membrane through a linker protein;
[11] The sugar chain-modified liposome according to [10] above, wherein the linker protein is a protein of biological origin;
[12] The sugar chain-modified liposome according to [11] above, wherein the linker protein is a protein of human origin;
[13] The sugar chain-modified liposome according to [12] above, wherein the linker protein is a serum protein of human origin;
[14] The sugar chain-modified liposome according to [11] above, wherein the linker protein is human serum albumin or bovine serum albumin;
[15] The sugar chain-modified liposome according to any one of [1] to [14] above, wherein the linker protein is coupled to a raft, which is composed of at least one type of lipid selected from the group consisting of gangliosides, glycolipids, phosphatidylglycerols, sphingomyelins and cholesterols, and which is formed on a surface of the liposome;
[16] The sugar chain-modified liposome according to any one of [1] to [15] above, which is hydrophilized by attaching a hydrophilic compound to the liposome membrane and/or a linker protein;
[17] The sugar chain-modified liposome according to [16] above, wherein the hydrophilic compound is a low-molecular-weight compound.
[18] The sugar chain-modified liposome according to [16] or [17] above, wherein the hydrophilic compound substantially does not sterically hinder the sugar chain and does not interfere with progress of a sugar chain molecule recognition reaction by lectin on a target cell membrane surface.
[19] The sugar chain-modified liposome of any one according to [16] to [18] above, wherein the hydrophilic compound has a hydroxyl group.
[20] The sugar chain-modified liposome of any one of [16] to [19] above, wherein the hydrophilic compound is an amino alcohol;
[21] The sugar chain-modified liposome of any one according to [16] to [20] above, wherein the hydrophilic compound is directly bonded to a surface of the liposome membrane;
[22] The sugar chain-modified liposome according to [16] above, which is hydrophilized by a hydrophilic compound, the hydrophilic compound being represented by general formula (1):

   X-R¹(R²OH)ₙ (1)

   wherein R¹ is a C₁ to C₄₀ linear or branched hydrocarbon chain, R² is either absent or a C₁ to C₄₀ linear or branched hydrocarbon chain, X is a reactive functional group which bonds either directly to a liposome lipid or a linker protein or to a crosslinking divalent reagent, and n is a positive integer;
[23] The sugar chain-modified liposome according to [16] above which is hydrophilized by a hydrophilic compound, the hydrophilic compound being represented by general formula (2):

   H_{2N}-R³-(R⁴OH)ₙ (2)

   wherein R³ is a C₁ to C₄₀ linear or branched hydrocarbon chain, R⁴ is either absent or a C₁ to C₄₀ linear or branched hydrocarbon chain, and n is a positive integer;
[24] The sugar chain-modified liposome according to [16] above, which is hydrophilized by a hydrophilic compound, the hydrophilic compound being represented by the general formula (3):

   H₂N-R⁵(OH)ₙ (3)

   wherein R⁵ is a C₁ to C₄₀ linear or branched hydrocarbon chain, and n is a positive integer;
[25] The sugar chain-modified liposome according to [16] above, wherein the liposome membrane and/or linker protein are hydrophilized by covalently bonding the hydrophilic compound, as a tris(hydroxyalkyl)aminoalkane, to the liposome membrane and/or the linker protein;
[26] The sugar-chain-modified liposome according to [25] above, wherein the liposome membrane and/or linker protein are hydrophilized by covalently bonding as a hydrophilic compound selected from the group consisting of tris(hydroxymethyl)aminoethane, tris(hydroxyethyl)aminoethane, tris(hydroxypropyl)aminoethane, tris(hydroxymethyl)aminomethane, tris(hydroxyethyl)aminomethane, tris(hydroxypropyl)aminomethane, tris(hydroxymethyl)aminopropane, tris(hydroxyethyl)aminopropane and tris(hydroxypropyl)aminopropane to the liposome membrane and/or the linker protein;
[27] The sugar chain-modified liposome according to any one of [1] to [26] above, wherein the sugar chain-modified liposome targets, as a receptor present on a cell membrane surface of various tissues, a lectin selected from the group consisting of C-type lectins including selectin, DC-SIGN, DC-SGNR, collectin and mannose-binding lectines, I-type lectins including Siglec, P-type lectins including mannose-6-phosphate receptors, R-type lectins, L-type lectins, M-type lectins and galectin;
[28] The sugar chain-modified liposome according to [27] above, wherein the sugar chain-modified liposome targets a selectin selected from the group consisting ofE-selectin, P-selectin and L-selectin;
[29] The sugar chain-modified liposome according to any one of [1] to [28] above, which has a sugar chain bonding density per liposome particle of from 1 to 30,000 sugar chains when a linker protein is used, and of from 1 to 500,000 sugar chains when a linker protein is not used;
[30] The sugar chain-modified liposome according to any one of [1] to [28] above, which includes a linker protein and has a sugar chain bonding density per linker protein molecule of from 1 to 60 sugar chains.
[31] The sugar chain-modified liposome according to any one of [1] to [30] above, wherein the liposome has an intestinal absorbability;
[32] The sugar chain-modified liposome according to any one of [1] to [31] above, which has a high targetability at a tissue or organ selected from the group consisting of blood, blood cells, liver, spleen, lungs, brain, small intestine, heart, thymus, kidneys, pancreas, muscle, large intestine, bones, bone marrow, eyes, ovaries, placenta, prostate, pituitary gland, mammary glands, blood vessels, skin, gall bladder, bile ducts, bladder, adipose tissue, cancer tissue, inflamed tissue and lymph nodes;
[33] The sugar chain-modified liposome according to any one of [1] to [32] above, wherein the sugar chain is selected from the group consisting of α-1,2-mannobiose disaccharide, α-1,3-mannobiose disaccharide, α-1,4-mannobiose disaccharide, α-1,6-mannobiose disaccharide, α-1,3-α-1,6-mannotriose trisaccharide, oligomannose-3 pentasaccharide, oligomannose-4b hexasaccharide, oligomannose-5 heptasaccharide, oligomannose-6 octasaccharide, oligomannose-7 nonasaccharide, oligomannose-8 decasaccharide, oligomannose-9 undecasaccharide, 3'-sialyllactose trisaccharide, 6'-sialyllactose trisaccharide, 3'-sialyllactosamine trisaccharide, 6'-sialyllactosamine trisaccharide, Lewis X trisaccharide, sialyl Lewis X tetrasaccharide, lactose disaccharide, 2'-fucosyllactose trisaccharide, difucosyllactose tetrasaccharide and 3-fucosyllactose trisaccharide;
[34] A liposomal preparation comprising the liposome according to any one of [1] to [33] above;
[35] The liposomal preparation according to [34] above, which is an oral preparation;
[36] The liposomal preparation according to [34] above, which is a parenteral preparation;
[37] A liposome comprising a membrane which is hydrophilized and to a surface of which sugar chains are not bonded, the liposome containing at least one selected from among aromatase inhibitors, anti-androgenic agents, lyase inhibitors, GnRH agonists, GnRH antagonists, anti-angiogenic agents, tyrosine kinase inhibitors, serine-threonine kinase inhibitors, antibodies having anticancer activities, ansamitocin, capecitabine, celmoleukin, docetaxel hydrate, gemcitabine hydrochloride, oxaliplatin, prednisolone, tegafur-uracil mixtures, zinostatin stimalamer and arsenic trioxide;
[37a] A liposome comprising a membrane which is hydrophilized and to a surface of which sugar chains are not bonded, which liposome contains docetaxel hydrate;
[38] The liposome according to [37] above, wherein constituent lipids in the liposome include phosphatidylcholines (molar ratio, 0 to 70%); phosphatidylethanolamines (molar ratio, 0 to 30%); one or more lipid selected from the group consisting of phosphatidic acids, long-chain alkyl phosphates and dicetyl phosphates (molar ratio, 0 to 30%); one or more lipid selected from the group consisting of gangliosides, glycolipids, phosphatidylglycerols and sphingomyelins (molar ratio, 0 to 40%); and cholesterols (molar ratio, 0 to 70%);
[39] The liposome according to [37] or [38] above, which includes a protein;
[40] The liposome according to any one of [37] to [39] above, which is hydrophilized by attaching a hydrophilic compound to the liposome membrane and/or a linker protein;
[41] The liposome according to [40] above, wherein the hydrophilic compound is a low-molecular-weight substance;
[42] The liposome according to [40] or [41] above, wherein the hydrophilic compound substantially does not sterically hinder the sugar chain and does not interfere with progress of a sugar chain molecule recognition reaction by lectin on a target cell membrane surface;
[43] The liposome according to any one of [40] to [42] above, wherein the hydrophilic compound has a hydroxyl group;
[44] The liposome according to any one of [40] to [43] above, wherein the hydrophilic compound is an amino alcohol;
[45] The liposome according to any one of [40] to [44] above, wherein the hydrophilic compound is directly attached to a surface of the liposome membrane;
[46] The liposome according to [40] above, wherein the low-molecular-weight hydrophilic compound has at least one hydroxyl group;
[47] The liposome according to [40] above, wherein the hydrophilic compound is represented by general formula (1):

   X-R¹(R²OH)ₙ (1)

   wherein R¹ is a C₁ to C₄₀ linear or branched hydrocarbon chain, R² is either absent or a C₁ to C₄₀ linear or branched hydrocarbon chain, X is a reactive functional group which bonds either directly to a liposome lipid or a linker protein or to a crosslinking divalent reagent, and n is a positive integer;
[48] The liposome according to [40] above, wherein the hydrophilic compound is represented by general formula (2):

   H₂N-R³-(R⁴OH)ₙ (2)

   wherein R³ is a C₁ to C₄₀ linear or branched hydrocarbon chain, R⁴ is either absent or a C₁ to C₄₀ linear or branched hydrocarbon chain, and n is a positive integer;
[49] The liposome according to [40] above, wherein the hydrophilic compound is represented by general formula (3):

   H₂N-R⁵(OH)ₙ (3)

   wherein R⁵ is a C₁ to C₄₀ linear or branched hydrocarbon chain, and n is a positive integer;
[50] The liposome of [40] above, wherein the liposome membrane and/or linker protein are hydrophilized by covalently bonding the hydrophilic compound, as a tris(hydroxyalkyl)aminoalkane, to the liposome membrane and/or the linker protein;
[51] The liposome according to [50] above, wherein the liposome membrane and/or linker protein are hydrophilized by covalently bonding a hydrophilic compound selected from the group consisting of tris(hydroxymethyl)aminoethane, tris(hydroxyethyl)aminoethane, tris(hydroxypropyl)aminoethane, tris(hydroxymethyl)aminomethane, tris(hydroxyethyl)aminomethane, tris(hydroxypropyl)aminomethane, tris(hydroxymethyl)aminopropane, tris(hydroxyethyl)aminopropane and tris(hydroxypropyl)aminopropane to the liposome membrane and/or the linker protein;
[52] A liposomal preparation comprising the liposome according to any one of [37] to [51] above;
[53] A liposomal preparation comprising the liposome according to [52] above, which is an oral preparation; and
[54] A liposomal preparation comprising the liposome according to [52] above, which is a parenteral preparation.

Sugar chain-modified liposomes containing an aromatase inhibitor, anti-androgenic agent, lyase inhibitor, GnRH agonist, GnRH antagonist, anti-angiogenic agent, tyrosine kinase inhibitor, serine-threonine kinase inhibitor, antibody having an anticancer activity, ansamitocin, capecitabine, celmoleukin, docetaxel hydrate, gemcitabine hydrochloride, oxaliplatin, prednisolone, tegafur-uracil mixture, zinostatin stimalamer or arsenic trioxide have an excellent targetability at a tissue or organ selected from the group consisting of blood, liver, spleen, lungs, brain, small intestine, heart, thymus, kidneys, pancreas, muscle, large intestine, bones, bone marrow, eyes, ovaries, placenta, prostate, pituitary gland, mammary glands, blood vessels, skin, gall bladder, bile ducts, bladder, adipose tissue, cancer tissue, inflamed tissue and lymph node, and may therefore be employed as preparations capable of efficiently delivering the foregoing drug. Because the sugar chain-modified liposome of the invention thus has a high targetability, the above drug can be effectively made to accumulate at a targeted tissue or organ. Therefore, the concentration of a drug at the site of a disease can be increased without increasing the systemic dose of the drug, enabling cancer growth to be efficiently suppressed. Moreover, because it is possible to reduce the dose of the drug administered to the body as a whole without lowering the drug concentration at the targeted tissue (e.g., cancer tissue) or organ, side effects due to the drug can be reduced without diminishing the therapeutic effects.

Also, intestinal absorption of the sugar chain-modified liposome of the invention can be controlled, enabling the drug to be utilized as, for example, preparations which are distributed to tissues within the body via the intestinal tract.

Moreover, liposomes which contain an aromatase inhibitor, anti-androgenic agent, lyase inhibitor, GnRH agonist, GnRH antagonist, anti-angiogenic agent, tyrosine kinase inhibitor, serine-threonine kinase inhibitor, antibody having an anticancer activity, ansamitocin, capecitabine, celmoleukin, docetaxel hydrate, gemcitabine hydrochloride, oxaliplatin, prednisolone, tegafur-uracil mixture, zinostatin stimalamer or arsenic trioxide are very stable within the body, even when sugar chains are not attached to the liposome. As a result, the drug has a longer half-life within the body, enabling the drug to be effectively delivered to the targeted tissue or organ. The liposome of the invention may thus be employed as a preparation which enables the above drugs to be effectively delivered to a targeted tissue or organ.

### BRIEF DESCRIPTION OF THE DRAWINGS

FIG. 1 is a schematic diagram illustrating the structure of a liposome to which is attached α-1,2-mannobiose disaccharide.
FIG. 2 is a schematic diagram illustrating the structure of a liposome to which is attached α-1,3-mannobiose disaccharide.
FIG. 3 is a schematic diagram illustrating the structure of a liposome to which is attached α-1,4-mannobiose disaccharide.
FIG. 4 is a schematic diagram illustrating the structure of a liposome to which is attached α-1,6-mannobiose disaccharide.
FIG. 5 is a schematic diagram illustrating the structure of a liposome to which is attached α-1,3-α-1,6-mannobiose trisaccharide.
FIG. 6 is a schematic diagram illustrating the structure of a liposome to which is attached oligomannose-3 pentasaccharide.
FIG. 7 is a schematic diagram illustrating the structure of a liposome to which is attached oligomannose-4b pentasaccharide.
FIG. 8 is a schematic diagram illustrating the structure of a liposome to which is attached oligomannose-5 heptasaccharide.
FIG. 9 is a schematic diagram illustrating the structure of a liposome to which is attached oligomannose-6 octasaccharide.
FIG. 10 is a schematic diagram illustrating the structure of a liposome to which is attached oligomannose-7 nonasaccharide.
FIG. 11 is a schematic diagram illustrating the structure of a liposome to which is attached oligomannose-8 decasaccharide.
FIG. 12 is a schematic diagram illustrating the structure of a liposome to which is attached an oligomannose-9 undecasaccharide chain.
FIG. 13 is a diagram showing the distributions of 13 types of liposome complexes in the blood 60 minutes after intravenous administration.
FIG. 14 is a diagram showing the distributions of 13 types of liposome complexes in the liver 60 minutes after intravenous administration.
FIG. 15 is a diagram showing the distributions of 13 types of liposome complexes in the spleen 60 minutes after intravenous administration.
FIG. 16 is a diagram showing the distributions of 13 types of liposome complexes in the lungs 60 minutes after intravenous administration.
FIG. 17 is a diagram showing the distributions of 13 types of liposome complexes in the brain 60 minutes after intravenous administration.
FIG. 18 is a diagram showing the distributions of 13 types of liposome complexes in cancer tissue 60 minutes after intravenous administration.
FIG. 19 is a diagram showing the distributions of 13 types of liposome complexes in the lymph nodes 60 minutes after intravenous administration.
FIG. 20 is a diagram showing the distributions of 13 types of liposome complexes in the thymus 60 minutes after intravenous administration.
FIG. 21 is a diagram showing the distributions of 13 types of liposome complexes in the heart 60 minutes after intravenous administration.
FIG. 22 is a diagram showing the distributions of 13 types of liposome complexes in the small intestine 60 minutes after intravenous administration.
FIG. 23 is a schematic diagram illustrating the structure of a liposome to which is attached 3'-sialyllactose trisaccharide.
FIG. 24 is a schematic diagram illustrating the structure of a liposome to which is attached 6'-sialyllactose trisaccharide.
FIG. 25 is a schematic diagram illustrating the structure of a liposome to which is attached 3'-sialyllactosamine trisaccharide.
FIG. 26 is a schematic diagram illustrating the structure of a liposome to which is attached 6'-sialyllactosamine trisaccharide.
FIG. 27 is a diagram showing the amounts in which 4 types of liposome complexes have moved into the blood 10 minutes after intestinal administration.
FIG. 28 is a diagram showing the amounts in which 4 types of liposome complexes have moved into the blood 10 minutes after intestinal administration.
FIG. 29 is a diagram showing the amounts in which 4 types of liposome complexes have moved into the blood 10 minutes after intestinal administration.
FIG. 30 is a diagram showing the amounts in which 4 types of liposome complexes have moved into the blood 10 minutes after intestinal administration.
FIG. 31 is a diagram showing the amounts in which 4 types of liposome complexes have moved into the blood 10 minutes after intestinal administration.
FIG. 32 is a schematic diagram of, as a control, a liposome to which is attached tris(hydroxymethyl)aminomethane.
FIG. 33 is a schematic diagram illustrating the structure of a liposome to which is attached Lewis X trisaccharide.
FIG. 34 is a schematic diagram illustrating the structure of a liposome to which is attached sialyl Lewis X tetrasaccharide.
FIG. 35 is a schematic diagram illustrating the structure of a liposome modified with lactose disaccharide.
FIG. 36 is a schematic diagram illustrating the structure of a liposome modified with 2'-fucosyllactose trisaccharide.
FIG. 37 is a schematic diagram illustrating the structure of a liposome modified with difucosyllactose tetrasaccharide.
FIG. 38 is a schematic diagram illustrating the structure of a liposome modified with 3-fucosyllactose trisaccharide.

The present invention is described more fully below.

The invention relates to a sugar chain-modified liposome have a sugar chain attached to a liposome membrane (also abbreviated herein as "sugar chain-modified liposome of the invention"), which liposome contains a drug having an anticancer activity such as an aromatase inhibitor, anti-androgenic agent, lyase inhibitor, GnRH agonist, GnRH antagonist, anti-angiogenic agent, tyrosine kinase inhibitor, serine-threonine kinase inhibitor, antibody having anticancer activity, ansamitocin, capecitabine, celmoleukin, docetaxel hydrate, gemcitabine hydrochloride, oxaliplatin, prednisolone, tegafur-uracil mixture, zinostatin stimalamer or arsenic trioxide. Here, the term "cancer" encompasses all neoplasms such as tumors and leukemia.

Examples of the "aromatase inhibitor" include anastrozole, letrozole, fadrozole and exemestane.

Examples of the "anti-androgenic agent" include bicaltamide and flutamide.

An example of the "lyase inhibitor" is ketoconazole.

Examples of the "GnRH agonist" include goserelin acetate and leuprorelin acetate.

Examples of the "GnRH antagonist" include cetrorelix, ganirelix, abarelix and ozarelix.

An example of the "anti-angiogenic agent" is fumagillin.

Examples of the tyrosine kinase inhibitor include gefitinib, erulotinib, imatinib mesilate, lapatinib, sunitinib and dasatinib.

An example of the "serine-threonine kinase inhibitor" is sorafenib.

Examples of "antibodies having an anticancer activity" include rituximab, bevacizumab, cetuximab, trastuzumab and pertuzumab.

The sugar chain-modified liposome of the invention encompasses both targeted liposomes having various sugar chains on the surface and intestinal absorption-controlled liposomes. In the present invention, "targeted" refers to the ability to specifically reach and be taken up at a target site such as a specific tissue or organ or a site of disease such as cancer when administered within the body. "Intestinal absorption-controlled" refers to the ability to be taken up within the body via the intestinal tract, i.e., having intestinal absorbability, and also the ability to control the rate, degree etc. of such take-up.

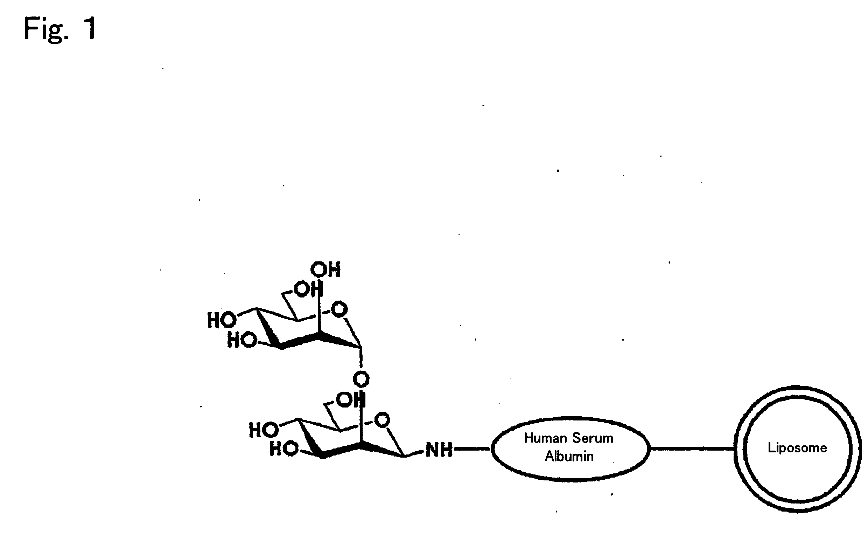

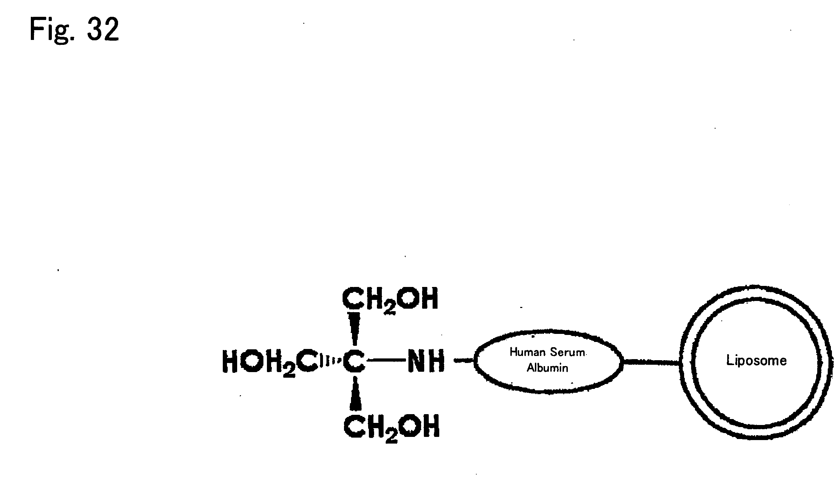

"Liposome" generally refers to a closed vesicle composed of a layer of lipids that have collected into the form of a membrane and a water layer at the interior. The sugar chain-modified liposomes of the invention, as shown in FIGS. 1 to 12, 23 to 26 and 32 to 38, have sugar chains attached to the surface thereof, i.e., the lipid layer. The sugar chains may be attached directly to the lipid layer of the liposome, or may be attached through a linker protein. The sugar chains are attached while incurring control over the type and density of the chains.

Various types of sugar chains may be used in accordance with the tissue, organ or cancer, etc. to be targeted by the intestinal adsorption-controlled liposomes of the invention and the targeted liposomes of the invention.

E-Selectin and P-selectin, which are expressed in the vascular endothelial cells of lesions, are known to bind strongly with sialyl Lewis X, which is a sugar chain expressed on the cell membrane of leukocytes. The above-mentioned liposomes are liposomes to which such sialyl Lewis X sugar chains or sugar chains which are similarly capable of reacting to a lectin-like (e.g., E-selectin, P-selectin) protein having sugar chain binding sites are attached under control of the sugar chain type and density, and are thought to accumulate specifically at cancer or other disease sites where vascular endothelial cells express E-selectin, P-selectin, etc. Also, sites of expression for E-selectin, P-selectin, etc. are sites where inflammation or angiogenesis have arisen; it is believed that the blood vessels at such sites have enlarged endothelial cell interstices, and that the liposomes which accumulate diffuse to the disease site and periphery thereof through such interstices. The diffused liposomes are taken up into various types of cells (endocytosis) at the disease site and periphery thereof, and the drug encapsulated within the liposomes is released inside the cells. The drug effects against cancer and other diseases are elicited by this type of mechanism.

The sugar chains used in this invention are described more fully below with regard to the liposomes and other elements.

### Sugar Chains

The sugar chains attached to the inventive liposome are exemplified by sugar chains capable of reacting with lectin-like proteins having sugar chain binding sites, such as the above-mentioned E-selectin, P-selectin, etc. "E-selectin, P-selectin, etc." refers herein to various types of lectins (sugar chain recognition proteins), including C-type lectins such as selectin (e.g., E-selectin, P-selectin, L-selectin), DC-SIGN, DC-SGNR, collectin and mannose-binding proteins, I-type lectins such as Siglec, P-type lectins such as mannose-6-phosphate receptors, R-type lectins, L-type lectins, M-type lectins and galectin. Because the cells to be targeted by the inventive liposomes differ in the types of lectin-like proteins having sugar chain-binding sites which are expressed by the cells, liposomes having a particular targetability to specific cells can be obtained by selecting the sugar chains according to the type of protein.

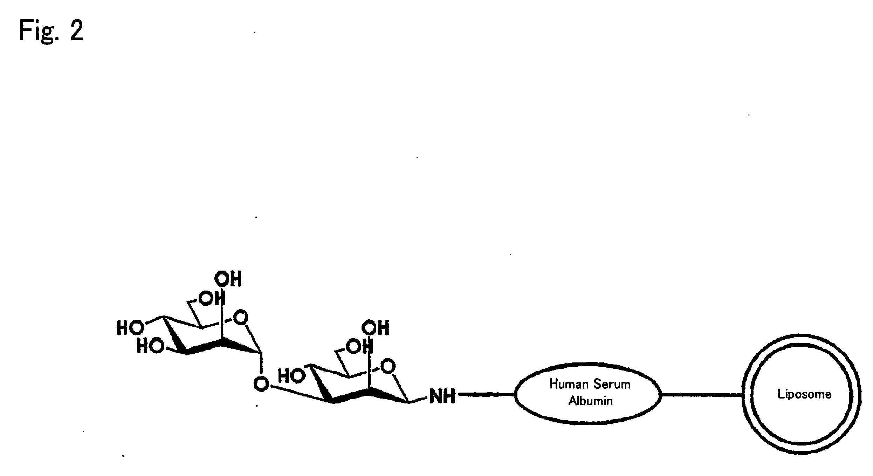

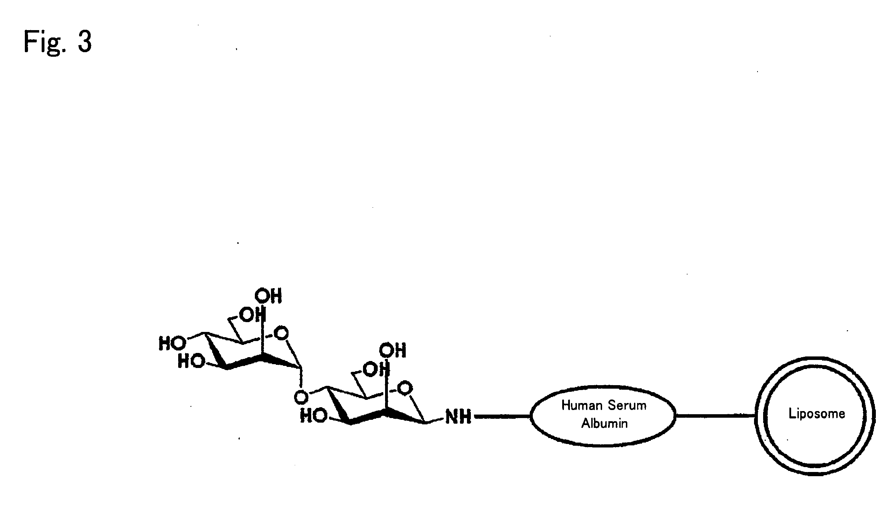

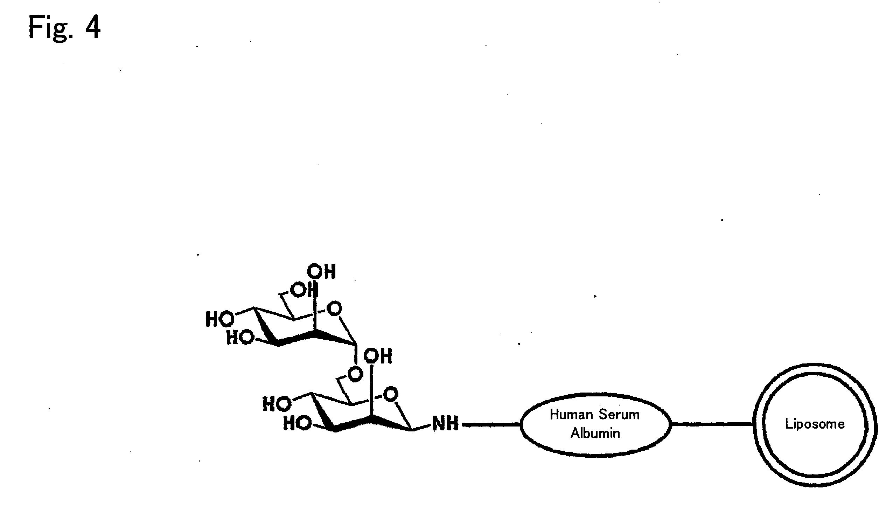

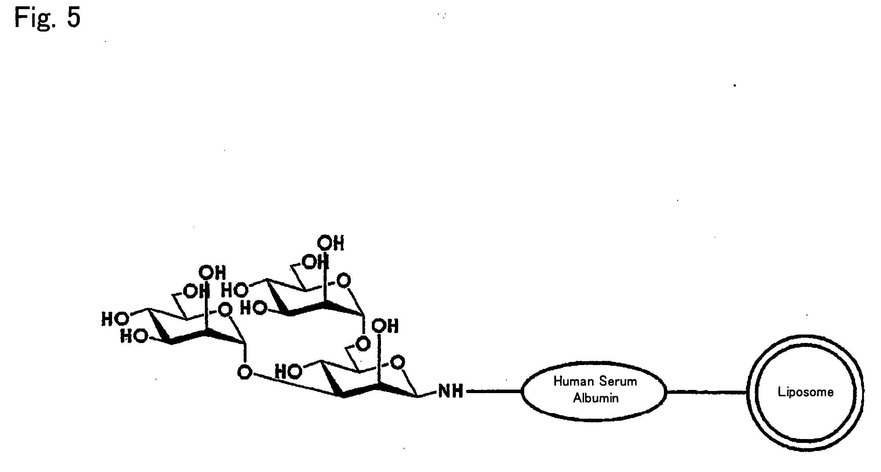

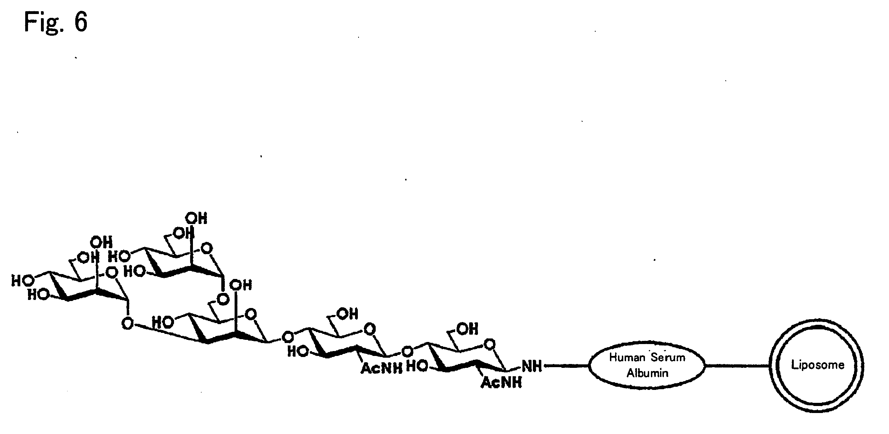

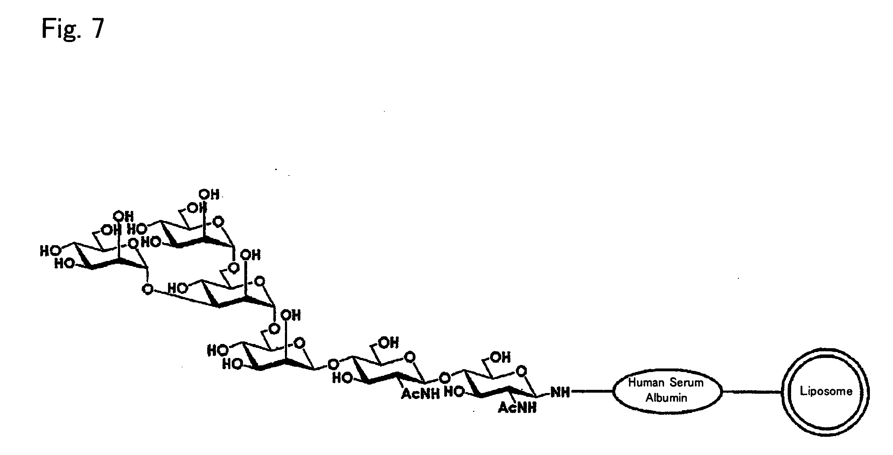

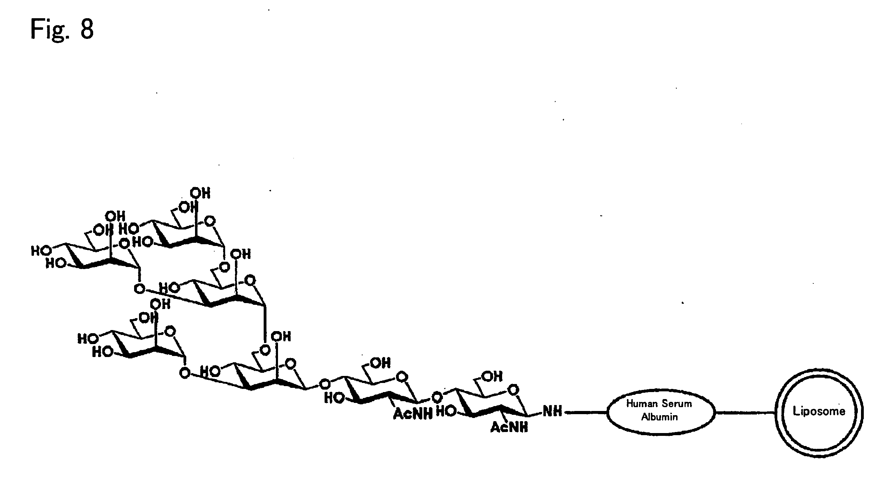

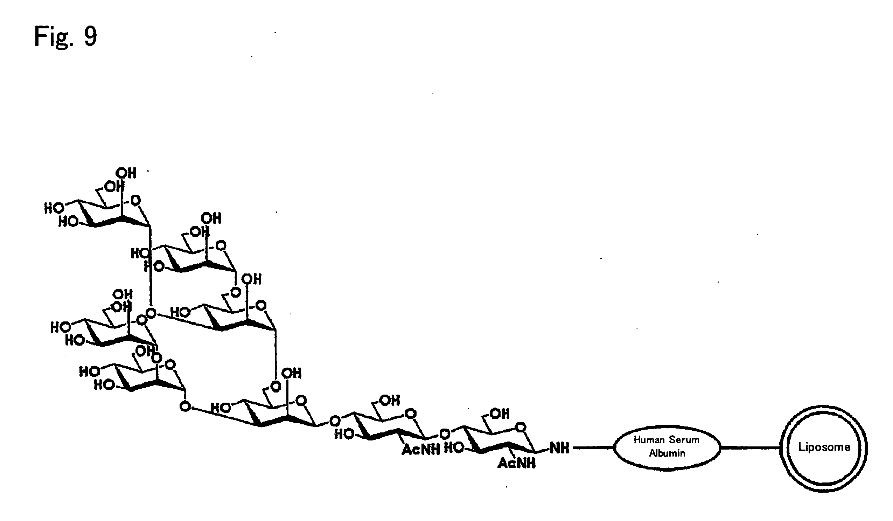

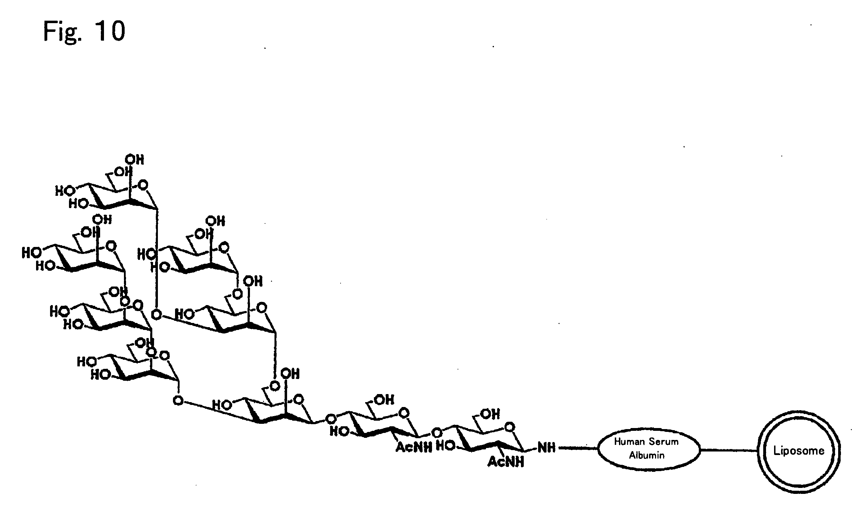

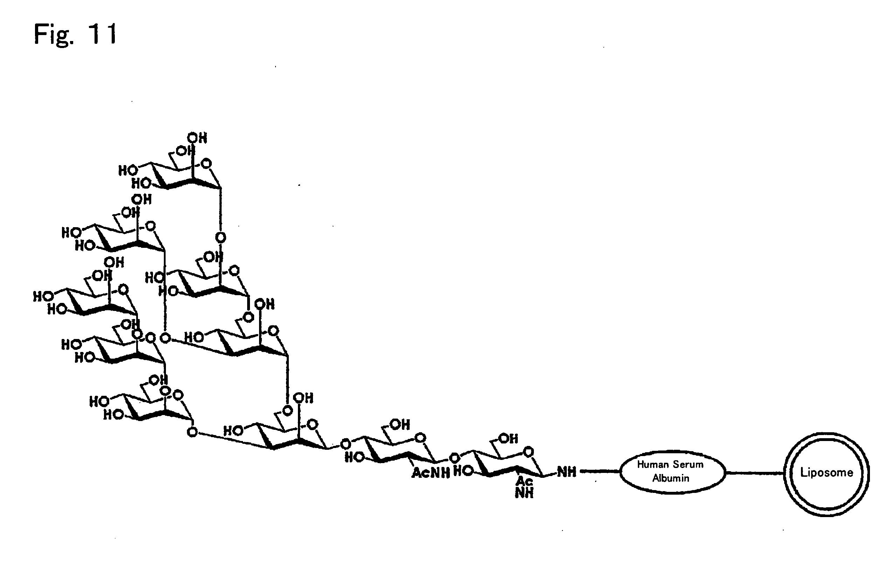

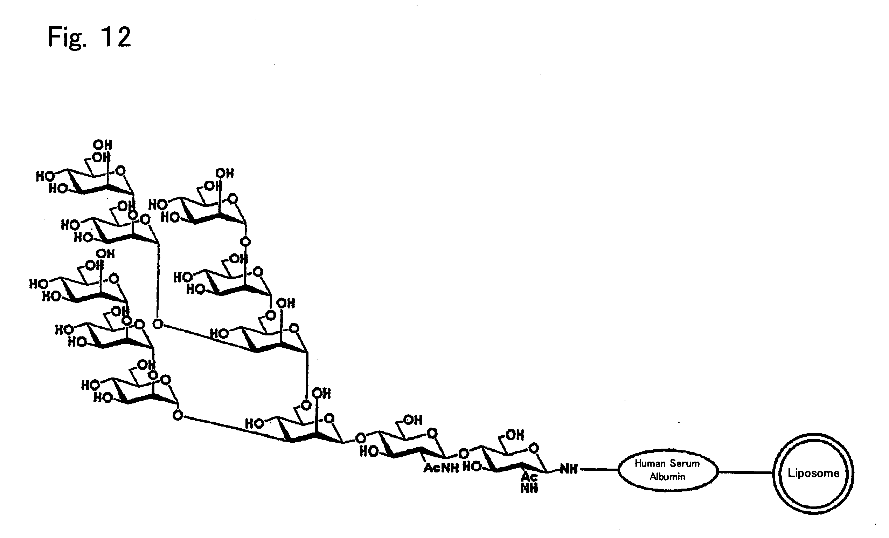

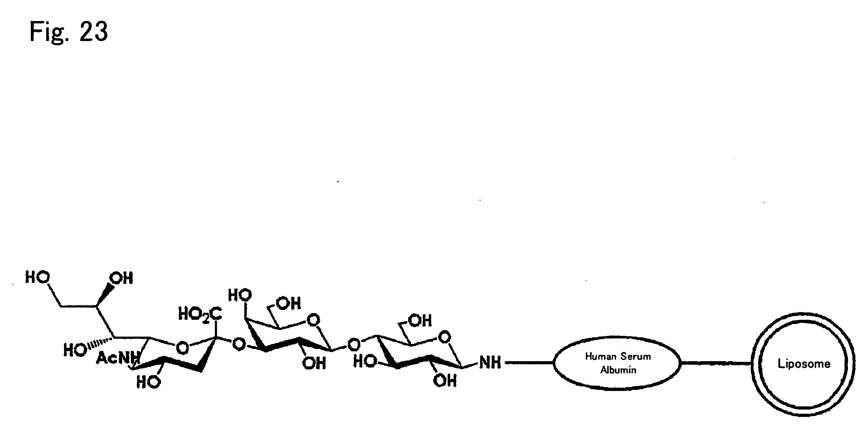

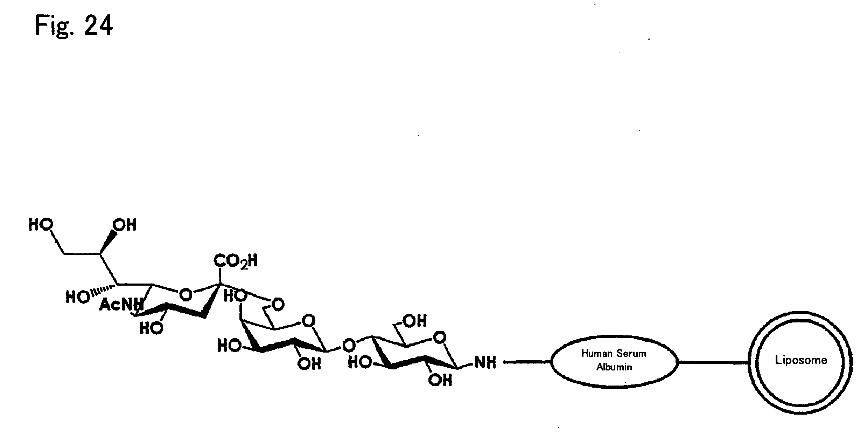

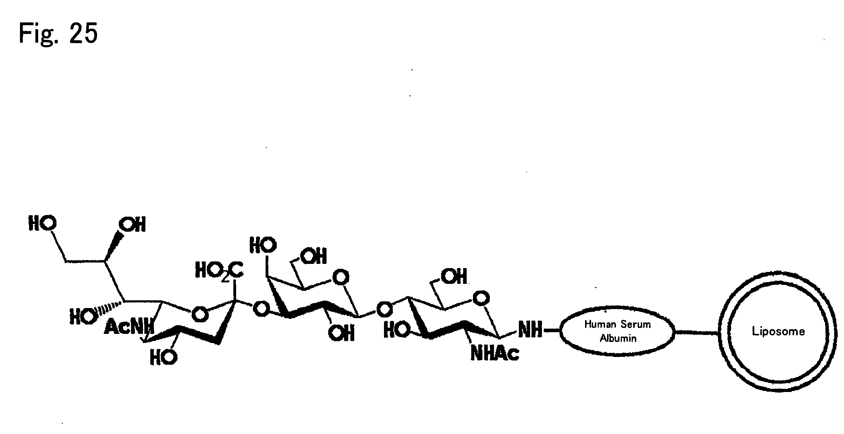

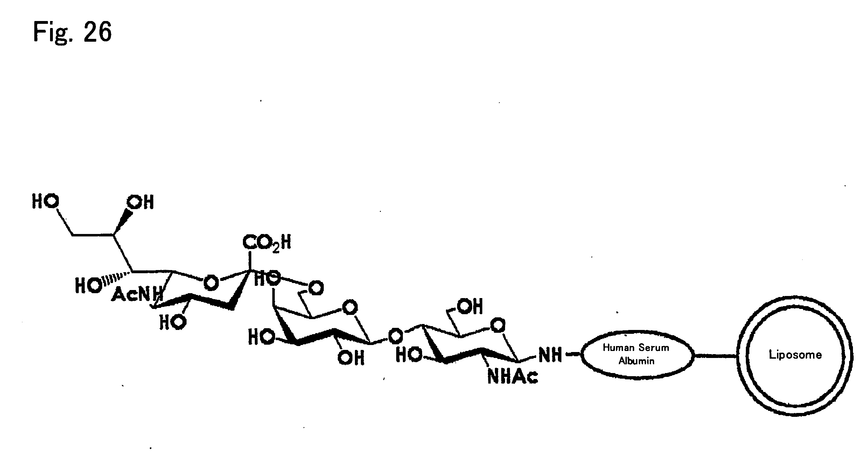

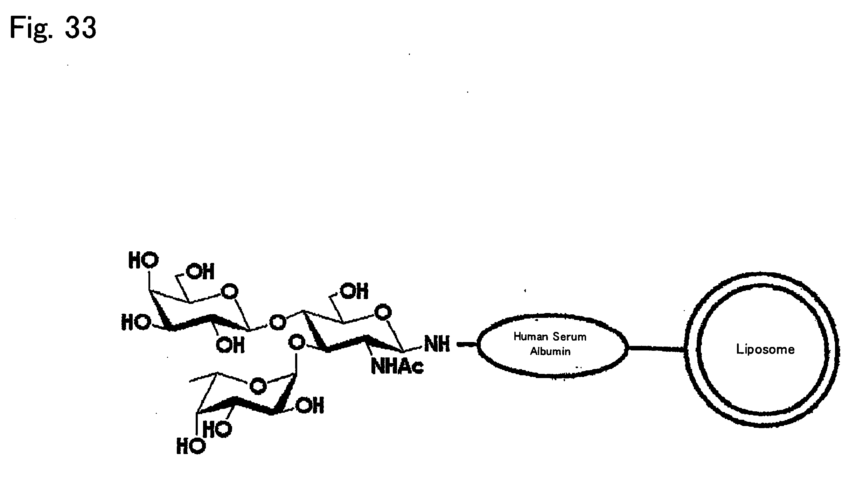

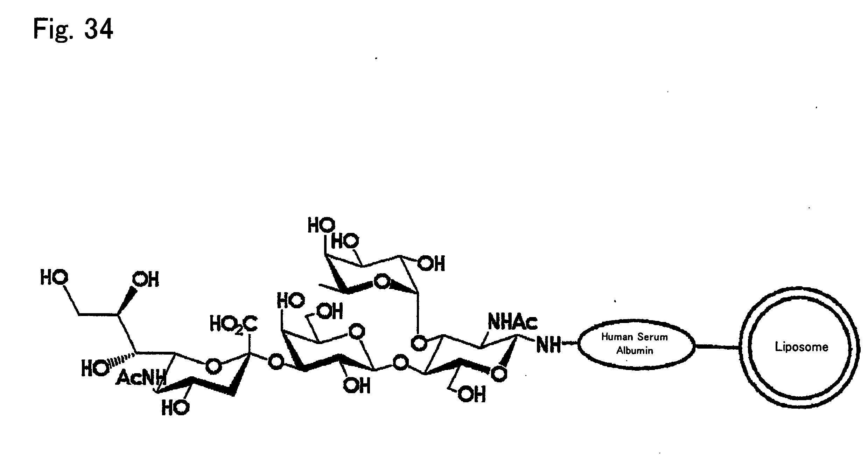

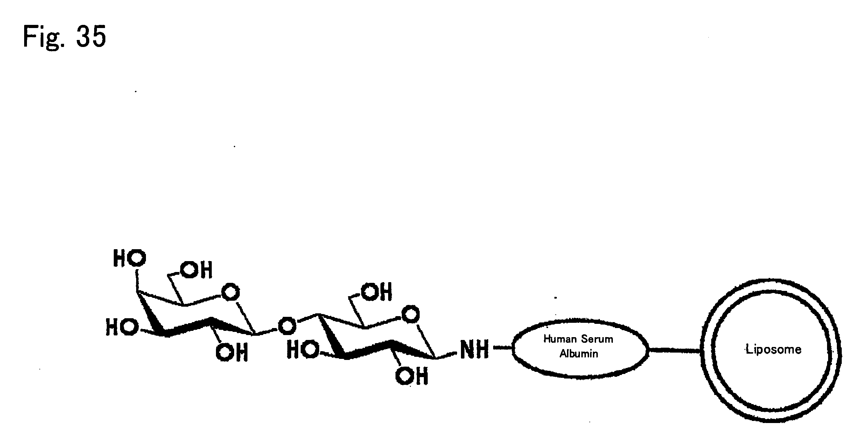

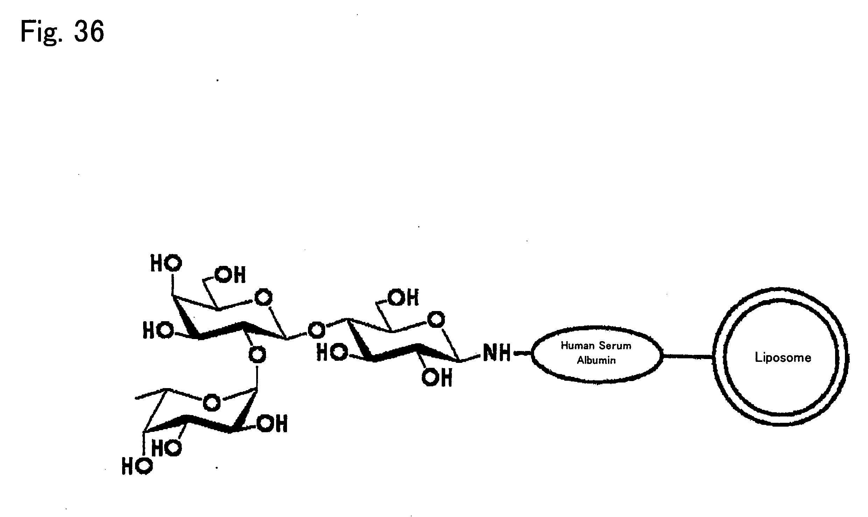

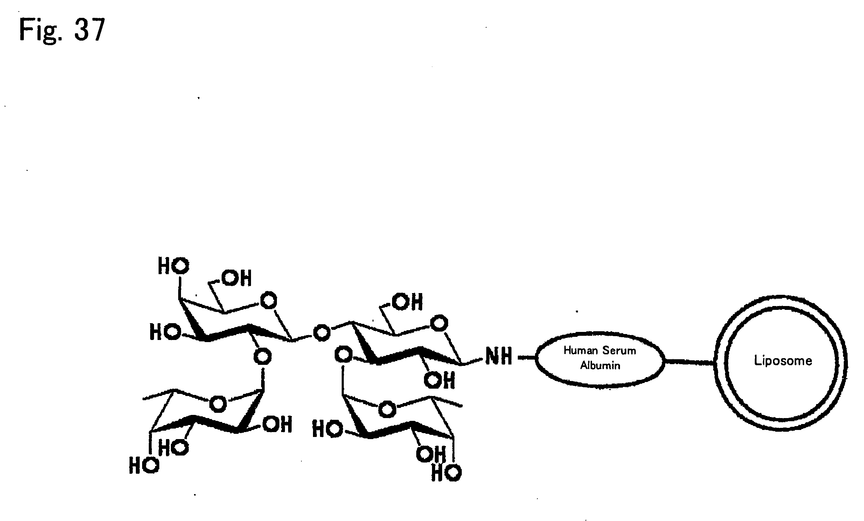

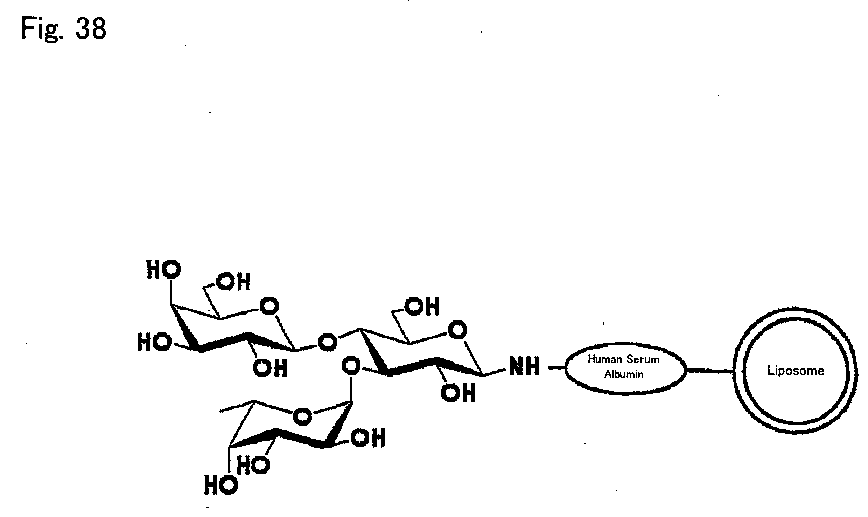

Examples of sugar chains that may be used in intestinal absorption-controlled liposomes include 3'-sialyllactose trisaccharide (the structural formula for which is shown in FIG. 23), 6'-sialyllactose trisaccharide (FIG. 24), 3'-sialyllactosamine saccharide (FIG. 25), and 6'-sialyllactosamine saccharide (FIG. 26). Examples of sugar chains that may be used in targeted liposomes include α-1,2-mannobiose disaccharide (FIG. 1), α-1,3-mannobiose disaccharide (FIG. 2), α-1,4-mannobiose disaccharide (FIG. 3), α-1,6-mannobiose disaccharide (FIG. 4), α-1,3-α-1,6-mannotriose trisaccharide (FIG. 5), oligomannose-3 pentasaccharide (FIG. 6), oligomannose-4b hexasaccharide (FIG. 7), oligomannose-5 heptasaccharide (FIG. 8), oligomannose-6 octasaccharide (FIG. 9), oligomannose-7 nonasaccharide (FIG. 10), oligomannose-8 decasaccharide (FIG. 11), oligomannose-9 undecasaccharide (FIG. 12), Lewis X trisaccharide (FIG. 33), sialyl Lewis X tetrasaccharide (FIG. 34), lactose disaccharide (FIG. 35), 2'-fucosyllactose trisaccharide (FIG. 36), difucosyllactose tetrasaccharide (FIG. 37) and 3-fucosyllactose trisaccharide (FIG. 38).

### Liposomes

Extensive efforts were made to construct, as the liposomes used in the invention, liposomes which are composed of various lipids and glycolipids such as those mentioned below in order to improve membrane stability, eliminate leakage of the encapsulated drug or the like, make it possible to hydrophilize the membrane surface, enable the binding of proteins at various densities, and enable the binding of sugar chains at various densities. The lipids that make up the inventive liposomes are exemplified by phosphatidylcholines, phosphatidylethanolamines, phosphatidic acids, long-chain alkyl phosphates, dicetyl phosphates, gangliosides, glycolipids, phosphatidylglycerols, sphingomyelins and cholesterols. Exemplary phosphatidylcholines include dimyristoylphosphatidylcholine, dipalmitoylphosphatidylcholine and distearoylphosphatidylcholine. Preferred exemplary phosphatidylethanolamines include dimyristoylphosphatidylethanolamine, dipalmitoylphosphatidylethanolamine and distearoylphosphatidylethanolamine. Preferred phosphatidic acids or long-chain alkyl phosphates include dimyristoylphosphatidic acid, dipalmitoylphosphatidic acid, distearoylphosphatidic acid and dicetyl phosphate. Preferred gangliosides include ganglioside GM1, ganglioside GD1a and ganglioside GT1b. Preferred glycolipids include galactosylceramide, glucosylceramide, lactosylceramide, phosphatide and globoside. Preferred phosphatidylglycerols include dimyristoylphosphatidylglycerol, dipalmitoylphosphatidylglycerol and distearoylphosphatidylglycerol. Of these, because phosphatidic acids or long-chain alkyl phosphates, gangliosides or glycolipids, and cholesterols have liposome stability-improving effects, their addition as the constituent lipid is desirable.

For example, the lipids that make up the inventive liposomes include phosphatidylcholines (molar ratio, 0 to 70%), phosphatidylethanolamines (molar ratio, 0 to 30%), one or more lipid selected from the group consisting of phosphatidic acids, long-chain alkyl phosphates and dicetyl phosphates (molar ratio, 0 to 30%), one or more lipid selected from the group consisting of gangliosides, glycolipids, phosphatidylglycerols and sphingomyelins (molar ratio, 0 to 40%), and cholesterols (molar ratio, 0 to 70%). The liposomes themselves may be produced according to a known method, illustrative examples of which include thin-film methods, reverse phase evaporation, ethanol injection and dehydration-rehydration.

In addition, by using such methods as sonication, extrusion, French pressing or homogenization, it is also possible to regulate the particle size of the liposomes. The method of producing the liposomes used in the invention involves first preparing mixed micelles of a lipid formulated from, for example, phosphatidylcholines, cholesterol, phosphatidylethanolamines, phosphatidic acids, gangliosides, glycolipids or phosphatidylglycerols in combination with the surfactant sodium cholate. The liposomes can then be created by carrying out ultrafiltration on the mixed micelles.

To negatively charge the liposomes, it is desirable to include a phosphatidic acid or a long-chain alkyl phosphate such as dicetyl phosphate. To provide hydrophilizing reaction sites, it is desirable to include a phosphatidylethanolamine. To provide linker protein binding sites, it is desirable to include a ganglioside, a glycolipid or a phosphatidylglycerol.

At least one type of lipid selected from the group consisting of gangliosides, glycolipids, phosphatidylglycerols, sphingomyelins and cholesterols collects together within the liposome and functions as a raft for binding linker proteins. The liposome of the invention is further stabilized by forming such a raft to which proteins can be bound. That is, the liposomes of the invention include liposomes in which has been formed a raft of at least one type of lipid selected from the group consisting of gangliosides, glycolipids, phosphatidylglycerols, sphingomyelines and cholesterols for binding linker proteins.

The liposome used in the invention may be an ordinary liposome, although it is desirable for the liposome surface to be hydrophilized. The liposome surface is hydrophilized following creation of the liposome in the above-described manner. Hydrophilization of the liposome surface is carried out by attaching a hydrophilic compound to the liposome surface. Examples of the compound used in hydrophilization include low-molecular-weight hydrophilic compounds, preferably low-molecular-weight hydrophilic compounds having at least one hydroxyl group, and more preferably low-molecular-weight hydrophilic compounds having at least two hydroxyl groups. Additional examples include low-molecular-weight hydrophilic compounds having also at least one amino group; that is, hydrophilic compounds having at least one hydroxyl group and at least one amino group on the molecule. Because such hydrophilic compounds have a low molecular weight, they substantially do not sterically hinder the sugar chain and do not interfere with progress of the sugar chain molecule recognition reaction by lectin on target cell membrane surfaces. It should be noted that the hydrophilic compounds do not include sugar chains which are used for orientation at specific targets such as lectin and are capable of bonding with the lectin. The hydrophilic compounds are exemplified by aminoalcohols such as tris(hydroxyalkyl)aminoalkanes (e.g., tris(hydroxymethyl)aminomethane). Specific examples include tris(hydroxymethyl)aminoethane, tris(hydroxyethyl)aminoethane, tris(hydroxypropyl)aminoethane, tris(hydroxymethyl)aminomethane, tris(hydroxyethyl)aminomethane, tris(hydroxypropyl)aminomethane, tris(hydroxymethyl)aminopropane, tris(hydroxyethyl)aminopropane and tris(hydroxypropyl)aminopropane. In addition, compounds obtained by introducing an amino group onto a low-molecular-weight compound having a hydroxyl group may also be used as the hydrophilic compound of the invention. Such compounds include, but are not limited to, compounds obtained by introducing an amino group onto a sugar chain which does not bond with lectin, such as cellobiose. For example, the liposome surface is hydrophilized by using a divalent reagent for crosslinking on the lipid phosphatidylethanolamine of the liposome membrane, and tris(hydroxymethyl)aminomethane. General formulas for the hydrophilic compound include formulas (1), (2) and (3) below.

X-R¹(R²OH)ₙ (1)

H₂N-R³-(R⁴OH)ₙ (2)

H₂N-R³(OH)ₙ (3)

Here, R¹, R³ and R⁵ are C₁ to C₄₀, preferably C₁ to C₂₀, and more preferably C₁ to C₁₀ linear or branched hydrocarbon chains; and R² and R⁴ are C₁ to C₄₀, preferably C₁ to C₂₀, and more preferably C₁ to C₁₀ linear or branched hydrocarbon chains. X is a reactive functional group which bonds directly with the liposome lipid or with a divalent reagent for crosslinking. Illustrative examples include COOH, NH, NH₂, CHO, SH, NHS-ester, maleimide, imidoester, active halogen, EDC, pyridyldisulfide, azidophenyl and hydrazide. The letter n is a positive integer.

The liposome surface that has been hydrophilized with such a hydrophilic compound is thinly covered with the hydrophilic compound. Because this hydrophilic compound covering has only a small thickness, even when sugar chains have been attached to the liposome, the reactivity of the sugar chains, etc. is not suppressed.

Hydrophilization of the liposome may be carried out by employing a method known to the art, such as the method of creating liposomes by using phospholipids to which polyethylene glycol, polyvinyl alcohol, maleic anhydride copolymer or the like are bound with covalent bonds (Japanese Patent Application Laid-open No. 2000-302685).

Of the above, it is especially preferable to hydrophilize the liposome surface using tris(hydroxymethyl)aminomethane.

Techniques according to the invention that involve the use of tris(hydroxymethyl)aminomethane are preferable in a number of ways to conventional hydrophilizing methods which use polyethylene glycol or the like. For example, with a method like that of the invention which attaches a sugar chain onto a liposome and utilizes a recognition function for that molecule in targeting, because the tris(hydroxymethyl)aminomethane is a low-molecular-weight substance, it is particularly desirable compared with conventional methods that use high-molecular-weight substances such as polyethylene glycol because it substantially does not sterically hinder the sugar chain and does not interfere with progress of the sugar chain molecule recognition reaction by lectin (sugar chain recognition protein) on a target cell membrane surface. That is, in the present specification, "low-molecular-weight substance or low-molecular-weight compound used in hydrophilization" refers to a substance which, when attached to the liposome surface, compared to high-molecular-weight substances such as polyethylene glycol, substantially does not sterically hinder the sugar chain and does not interfere with progress of the sugar chain molecule recognition reaction by lectin (sugar chain recognition protein) on a target cell membrane surface.

Because the liposomes of the invention, even after such hydrophilizing treatment, have a good particle size distribution, ingredient composition and dispersion properties, and also have a long shelf-life and an excellent stability in the body, they are desirable for use in the production of liposomal preparations.

The liposome surface may be hydrophilized as follows using tris(hydroxymethyl)aminomethane. A divalent reagent such as bis(sulfosuccinimidyl) suberate, disuccinimidyl glutarate, dithiobis(succinimidyl propionate), disuccinimidyl suberate, 3,3'-dithiobis(sulfosuccinimidyl propionate), ethylene glycol bis(succinimidyl) succinate and ethylene glycol bis(sulfosuccinimidyl) succinate is added to a liposome solution obtained by a conventional method using a lipid such as dimyristoylphosphatidylethanolamine, dipalmitoylphosphatidylethanolamine or distearoylphosphatidylethanolamine and the reaction is carried out, thereby binding the divalent reagent to the lipid such as dipalmitoylphosphatidylethanolamine on the liposome membrane. Next, tris(hydroxymethyl)aminomethane is reacted with one of the valence bonds on the divalent reagent, thereby attaching tris(hydroxymethyl)aminomethane to the liposome surface.

Hydrophilized liposomes obtained in this way are very stable in the body and, even without bonding sugar chains having targetability as subsequently described, have a long half-life in the body, enabling them to be suitably used as drug carriers in drug delivery systems. The present invention also encompasses liposomes whose surfaces have been hydrophilized with a low-molecular-weight compound.

The present invention additionally encompasses liposomes which have been hydrophilized using the above-described hydrophilic compound and to which sugar chains are not attached. In such hydrophilized liposomes, the liposome itself has a high stability; moreover, when sugar chains are attached, the hydrophilized liposome has an increased sugar chain recognizability.

The liposomes used in the invention are liposomes wherein the constituent lipids include phosphatidylcholines (molar ratio, 0 to 70%); phosphatidylethanolamines (molar ratio, 0 to 30%); one or more lipid selected from the group consisting of phosphatidic acids, long-chain alkyl phosphates and dicetyl phosphates (molar ratio, 0 to 30%); one or more lipid selected from the group consisting of gangliosides, glycolipids, phosphatidylglycerols and sphingomyelins (molar ratio, 0 to 40%); and cholesterols (molar ratio, 0 to 70%).

The present invention also encompasses a method of hydrophilizing liposomes by attaching the above-described hydrophilic compound to the liposome, and additionally encompasses hydrophilized liposomes to which sugar chains are not attached ("Liposomes to which sugar chains are not attached" is sometimes abbreviated below as "the liposomes of the invention." "Hydrophilicized liposomes to which sugar chains are not attached" and "sugar chain-modified liposomes of the invention" are also sometimes referred to collectively as "the liposomes of the invention."). By attaching sugar chains to a liposome to which sugar chains are not attached, the targeted liposomes or intestinally absorbable liposomes of the invention can be produced.

The liposomes of the invention are very stable, enabling post-treatment such as the coupling of protein, the coupling of linker protein or the attachment of sugar chains after liposome formation. Therefore, after a large amount of liposomes has been produced, by binding, according to the intended purpose, various different proteins, linker proteins and sugar chains, it is possible to produce a variety of liposomes for the intended purposes.

Sugar chains are attached to the liposomes of the invention either through a linker protein or directly to the lipids making up the liposome. The liposomes of the invention are preferably liposomes which have complex carbohydrate ligands such as glycolipids or glycoproteins and have been hydrophilized with low-molecular-weight compounds.

In the liposomes of the invention, the particle size of the liposomes having sugar chains and the like attached thereto is from 30 to 500 nm, and preferably from 50 to 350 nm. It is desirable for the liposomes of the invention to be negatively charged. By being negatively charged, interactions with negatively charged cells in the body can be prevented. The zeta potential at the surface of the liposomes of the invention, in physiological saline and at 37°C, is from -50 to 10 mV, preferably from -40 to 0 mV, and more preferably from -30 to -10 mV. As noted above, by including phosphatidic acids or long-chain alkyl phosphates such as dicetyl phosphate, the liposomes can be negatively charged.

The particle size and zeta potential can be measured using an apparatus for measuring zeta potential, analyzing particle size and measuring molecular weight (Model Nano ZS; Malvern Instruments Ltd., UK).

### Attachment of Sugar Chains to Liposomes

In the present invention, any of the above-mentioned sugar chains may be directly attached to the liposomes produced as described above, or any of the sugar chains may be attached through linker proteins. The types of sugar chains attached to the liposomes at this time are not limited to one type only; it is possible for a plurality of sugar chain types to be attached. The plurality of sugar chains in the latter case may be a plurality of sugar chains having binding activities to different lectins which are present together at the surfaces of cells in the same tissue or organ, or may be sugar chains having binding activities to different lectins present at the surfaces of cells in different tissues or organs. Selecting a plurality of sugar chains in the former of these two cases enables a specific target tissue or organ to be reliably targeted. Selecting a plurality of sugar chains in the latter of these two cases enables one type of liposome to be directed at a plurality of targets, thus enabling multipurpose targeted liposomes to be obtained.

Attachment of the sugar chains to the liposomes may be achieved by mixing linker protein and/or sugar chains at the time of liposome production so that the sugar chains are made to attach to the surfaces of the liposomes as the liposomes are being produced. However, it is more desirable to separately furnish the liposome, linker protein and sugar chains, and to couple the linker protein and/or sugar chains to the liposomes once liposome production is complete. The reason is that, by coupling linker proteins and/or sugar chains to the liposomes, the density of the sugar chains to be attached can be controlled.

Direct attachment of the sugar chains to the liposome may be carried out by a method such as that described below.

The sugar chain density is controlled either at the time of liposome production following the mixture of sugar chains as glycolipids, or when the sugar chains are attached to liposome phospholipids following liposome production.

When the sugar chains are attached using a linker protein, it is preferable to use a protein of biological origin, and in particular a protein of human origin. Proteins of biological origin include, but are not limited to, albumin and other proteins present in the blood, and other physiologically active substances present in organisms. Examples include human serum albumin (HSA) and animal serum albumins such as bovine serum albumin (BSA). In cases where human serum albumin in particular is used, it has been verified by experiments on mice that uptake in various tissues is high. It is possible to use a protein having an anticancer activity as the linker protein. In such a case, the protein may serve both as a linker protein for binding together liposomes and sugar chains and also as a protein having an anticancer activity. Proteins having an anticancer activity are exemplified by antibodies having anticancer activities.

The linker protein is preferably coupling to a raft which is composed of at least one type of lipid selected from the group consisting of gangliosides, glycolipids, phosphatidylglycerols, sphingomyelins and cholesterols and is formed on the liposome surface.

Attachment of the sugar chain to a liposome through a linker protein may be carried out by the method described below.

First, the protein is coupled to the liposome surface. The liposome is treated with an oxidizing agent such as NaIO₄, Pb(O₂CCH₃)₄ or NaBiO₃, thereby oxidizing the ganglioside present at the liposome membrane surface. Next, using a reagent such as NaBH₃CN or NaBH₄, the linker protein and the ganglioside on the liposome membrane surface are coupled by a reductive amination reaction. It is preferable to hydrophilize this linker protein as well, this being done by attaching a compound having a hydroxyl group to the linker protein. For example, this may be done by using a divalent reagent such as bis(sulfosuccinimidyl) suberate, disuccinimidyl glutarate, dithiobis(succinimidyl propionate), disuccinimidyl suberate, 3,3'-dithiobis(sulfosuccinimidyl propionate), ethylene glycol bis(succinimidyl) succinate or ethylene glycol bis(sulfosuccinimidyl) succinate to bond the above-described compound used in hydrophilization (e.g., tris(hydroxymethyl)aminomethane) to the linker protein on the liposome.

Specifically, first one end of the divalent reagent for crosslinking is bonded to all the amino groups on the linker proteins. Next, sugar chain-glycosylamine compounds obtained by glycosylaminating the reducing ends of the various sugar chains are prepared, and the amino groups of these sugar chains are bonded with the other unreacted end on a portion of the crosslinking divalent reagent that has been attached as described above onto the liposomes.

It is also possible to cleave the covalent bond between the sugar chain and/or hydrophilic compound and the liposome, or the covalent bond between the sugar chain and/or the hydrophilic compound and the linker protein, when the liposome is taken up by a cell. For example, in cases where the linker protein and the sugar chain are covalently bonded through a disulfide linkage, they undergo reduction within the cell and the sugar chain is cleaved. Cleavage of the sugar chain renders the liposome surface hydrophobic, allowing the liposome to bind with a biomembrane. This disrupts the stability of the liposome membrane, resulting in release of the drug contained within the liposome.

Next, hydrophilizing treatment is carried out using most of the unreacted ends on the divalent reagent to which sugar chains are not attached and which remains unreacted at the surface of the sugar chain-modified liposome membrane thus obtained. That is, the entire surface of the liposome is hydrophilized by carrying out a binding reaction between the unreacted end of the divalent reagent attached to protein on this liposome and the compound used in the above-described hydrophilization (e.g., tris(hydroxymethyl)aminomethane).

Hydrophilization of the liposome surface and the linker protein enhances the ability of the liposomes to move to various types of tissue, and also improves the retention of liposomes within the blood and the ability of such liposomes to move from the blood to various tissues. This is probably because, due to the hydrophilization of the liposome surface and the linker protein surface, portions of the liposome complex other than the sugar chains are mistaken in various tissues for body water and therefore are not recognized by non-target tissue, etc., as a result of which only the sugar chains are recognized by the lectin (sugar chain recognition protein) of the target tissue.

Next, the sugar chain is attached to a linker protein on the liposome. This is done by using an ammonium salt such as NH₄HCO₃ or NH₂COONH₄ to glycosylaminate the reducing ends of the sugar chains, then using a divalent reagent such as bis(sulfosuccinimidyl) suberate, disuccinimidyl glutarate, dithiobis(succinimidyl propionate), disuccinimidyl suberate, 3,3'-dithiobis(sulfosuccinimidyl propionate), ethylene glycol bis(succinimidyl) succinate and ethylene glycol bis(sulfosuccinimidyl) succinate to bond the linker protein coupled to the liposome membrane surface with the glycosylaminated carbohydrate, thereby giving liposomes such as those shown in FIGS. 1 to 12, 23 to 26 and 33 to 38. These sugar chains are commercially available.

The sugar chain bonding density when sugar chains are attached is from 1 to 60, preferably from 1 to 40, and more preferably from 1 to 20, per molecule of linker protein attached to the liposome. When a linker protein is used, the sugar chain bonding density per liposome particle is from 1 to 30,000, preferably from 1 to 20,000, and more preferably from 1 to 10,000; from 100 to 30,000, preferably from 100 to 20,000, and even more preferably from 100 to 10,000; or from 500 to 30,000, preferably from 500 to 20,000, and more preferably from 500 to 10,000. When a linker protein is not used, a maximum of 1 to 500,000, preferably from 1 to 300,000, and even more preferably from 1 to 100,000 or more, sugar chains may be attached to the liposome.

In the present invention, the targetability to various target cells and tissues can be controlled by variously selecting the sugar chain structure used and the amount of sugar chains attached.

Depending on the type and amount of sugar chains attached, it is also possible to increase the absorption controllability in the intestinal tract. By attaching to the liposomes both sugar chains which increase the intestinal absorption controllability and sugar chains having a targetability to a specific tissue or organ, it is possible to produce liposomes which are both targeted at a specific tissue or organ and also have an intestinal absorption controllability.

As explained above with regard to the targeted liposomes of the invention, the lectins to which the sugar chains specifically bind are determined by the type and amount of sugar chains attached to the liposome, resulting in delivery of the liposomes to a specific tissue or organ. Moreover, by selecting the sugar chain structure and the amount of sugar chains attached, delivery to disease sites such as cancer tissue is possible.

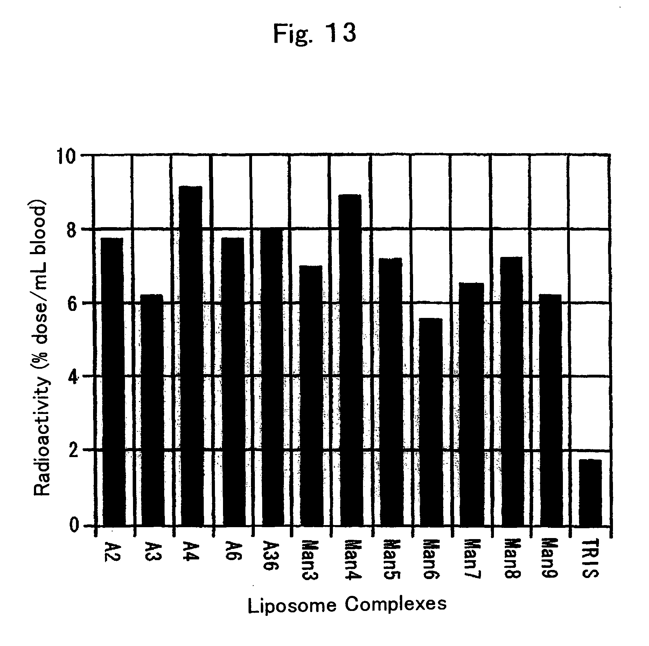

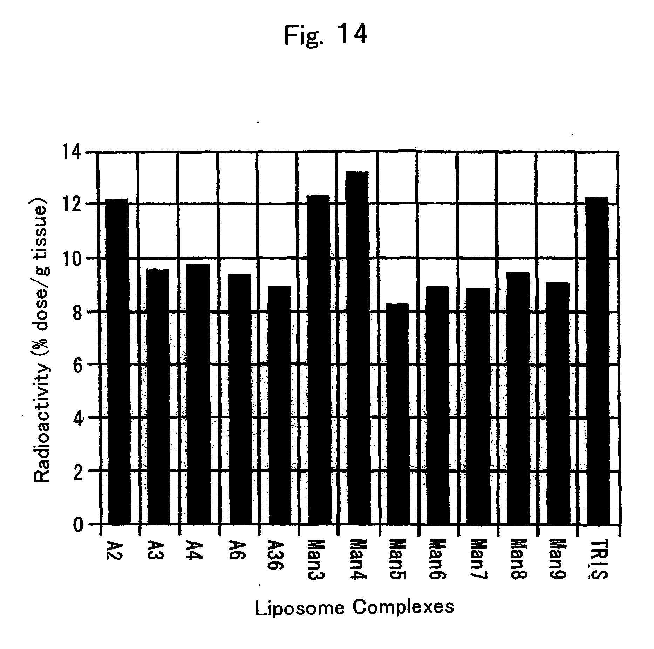

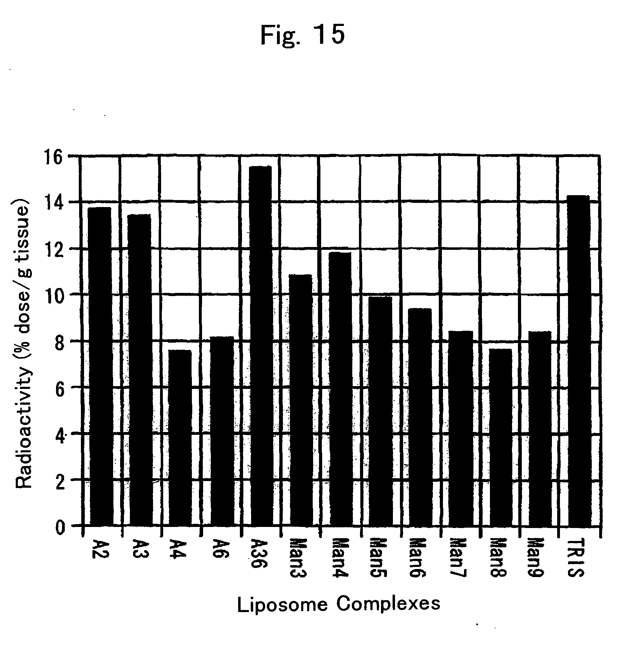

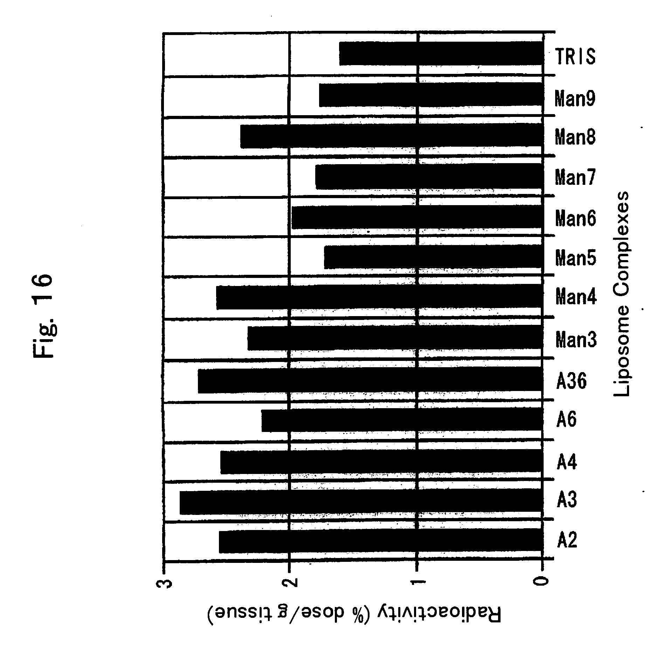

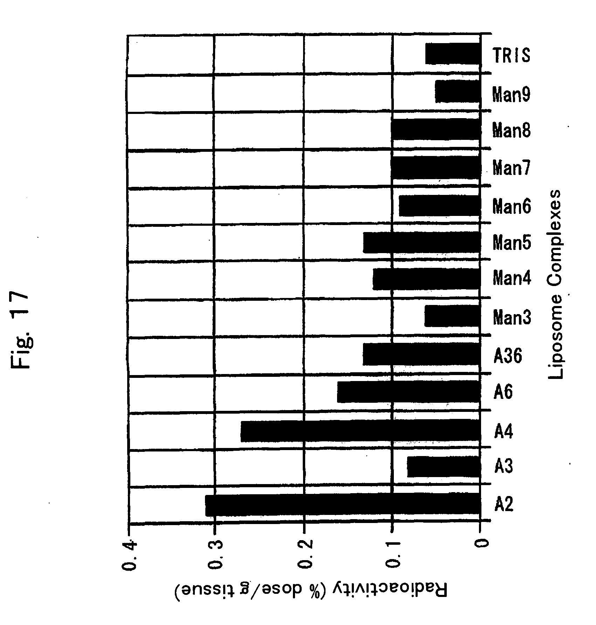

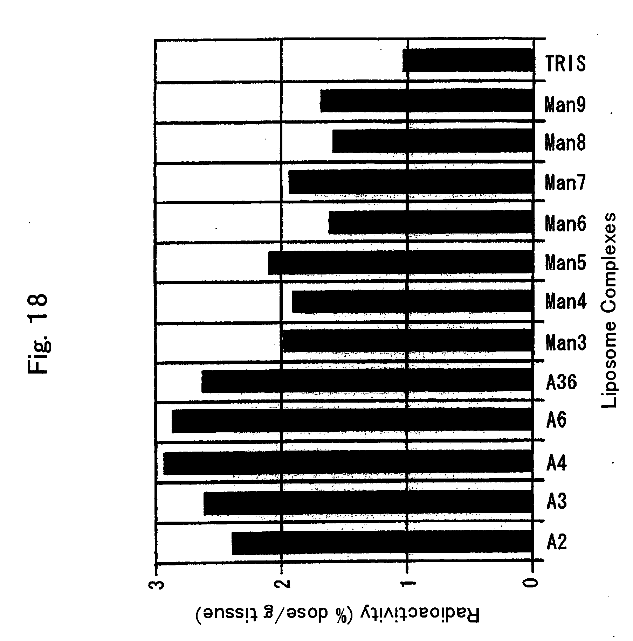

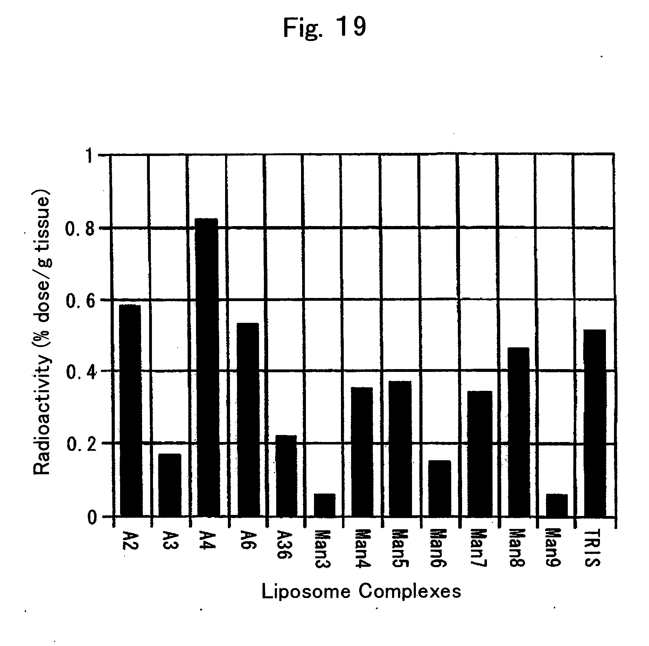

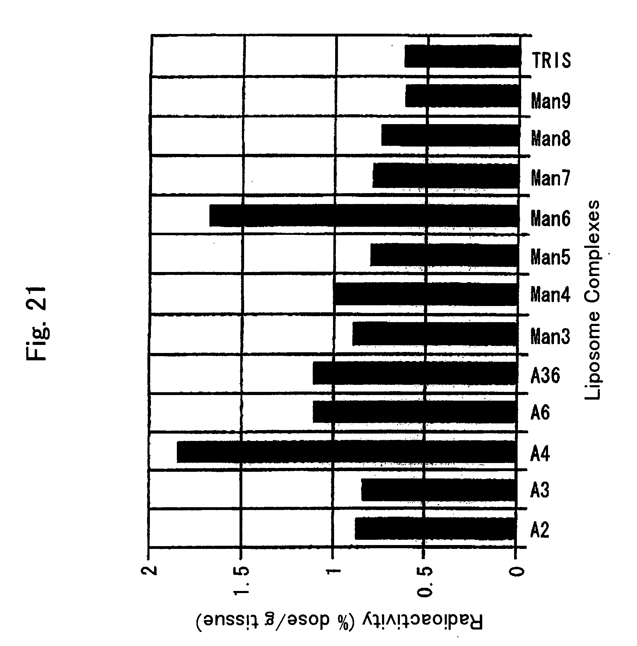

In the present invention, by judicious selection of the structure of the sugar chains used and the amount of sugar chains attached, it is possible to control the targetability of the liposome to various target cells and tissues. The sugar chain-modified liposomes of the invention are directed by means of the sugar chains to tissues or organs such as blood, liver, spleen, lungs, brain, small intestine, heart, thymus, kidneys, pancreas, muscle, large intestine, bones, bone marrow, eyes, ovaries, placenta, prostate, pituitary gland, mammary glands, blood vessels, skin, gall bladder, bile ducts, bladder, adipose tissue, cancer tissue, inflamed tissue and lymph nodes. For example, as shown in FIGS. 13, 16, 17, 18, 21 and 22, liposomes to which are attached α-1,2-mannobiose disaccharide, α-1,3-mannobiose disaccharide, α-1,4-mannobiose disaccharide, α-1,6-mannobiose disaccharide, α-1,3-α-1,6-mannotriose trisaccharide, oligomannose-3 pentasaccharide, oligomannose-4b hexasaccharide, oligomannose-5 heptasaccharide, oligomannose-6 octasaccharide, oligomannose-7 nonasaccharide, oligomannose-8 decasaccharide or oligomannose-9 undecasaccharide all have high targetabilities to the blood, lungs, brain, cancer tissue, heart and small intestine. As shown in FIG. 14, liposomes to which are attached α-1,2-mannobiose disaccharide, oligomannose-3 pentasaccharide or oligomannose-4b hexasaccharide have a high targetability to the liver. As shown in FIG. 15, liposomes to which are attached α-1,2-mannobiose disaccharide, α-1,3-mannobiose disaccharide or α-1,3-α-1,6-mannotriose trisaccharide have a high targetability to the spleen. As shown in FIG. 19, liposomes to which are attached α-1,2-mannobiose disaccharide, α-1,4-mannobiose disaccharide or α-1,6-mannobiose disaccharide have a high targetability to the lymph nodes. In addition, liposomes to which are attached oligomannose-6 octasaccharide have a high targetability to the thymus.

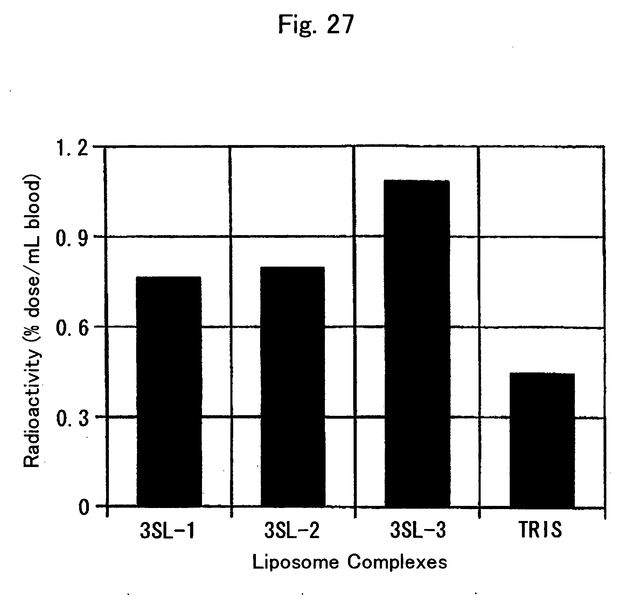

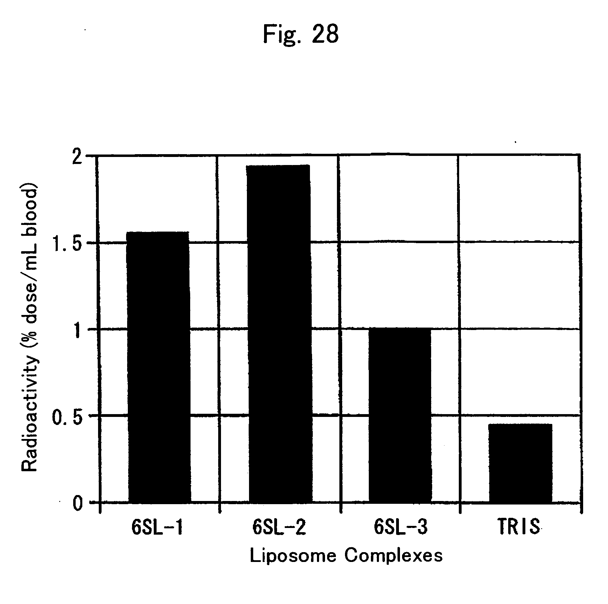

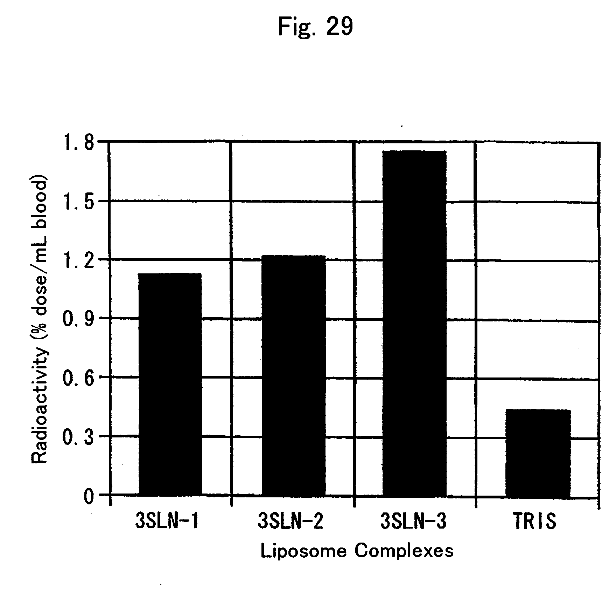

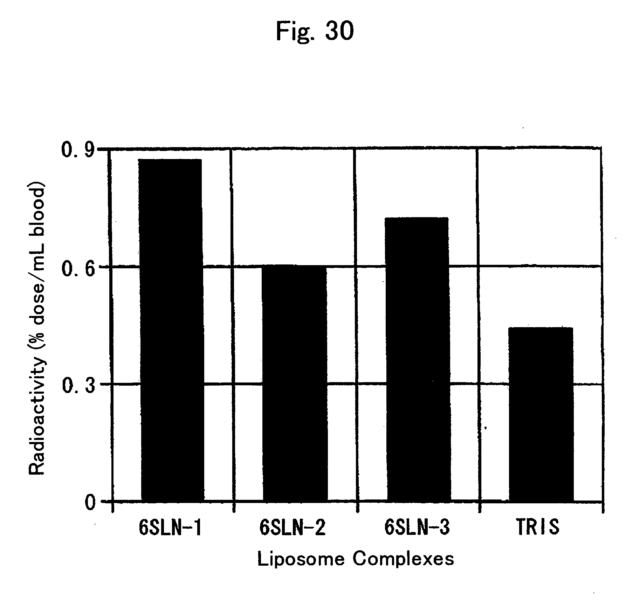

Also, the liposomes of the invention shown in FIGS. 23 to 26 generally have a very high intestinal absorbability. However, by adjusting the density of sugar chains on the liposome, it is possible to control the intestinal absorption and induce more efficient movement of the drug to target sites, enabling the mitigation of side effects. For example, in FIGS. 27 to 30, an ability to move from the intestinal tract into the blood (i.e., intestinal absorbability) is exhibited when the amount of sugar chains attached in the four above types of sugar chain-modified liposomes is varied over three levels.

This variation in the amount of attached sugar chains was carried out by attaching the sugar chains to the linker protein-coupled liposome at three concentrations (i.e., 1) 50 µg, 2) 200 µg, 3) 1 mg). In the case of 6'-sialyllactose trisaccharide and 6'-sialyllactosamine saccharide, as the sugar chain density is increased, the intestinal absorption gradually decreases. In the case of 3'-sialyllactose trisaccharide and 3'-sialyllactosamine saccharide, the reverse occurs; i.e., the intestinal absorption increases. These findings indicate that the intestinal absorption differs for each type of sugar chain, depending on the amount of sugar chains which are attached to the liposome. Therefore, by suitably selecting the amount of sugar chains which are attached to the liposome for each type of sugar chain, it is possible to control the intestinal absorption.

### Drug-Containing Liposomes and Liposomal Preparations

The liposomes of the invention contain a drug having an anticancer activity, such as an aromatase inhibitor, an anti-androgenic agent, a lyase inhibitor, an GnRH agonist, an GnRH antagonist, an anti-angiogenic agent, a tyrosine kinase inhibitor, a serine-threonine kinase inhibitor, an antibody having an anticancer activity, ansamitocin, capecitabine, celmoleukin, docetaxel hydrate, gemcitabine hydrochloride, oxaliplatin, prednisolone, a tegafur-uracil mixture, zinostatin stimalamer or arsenic trioxide. The liposomes are delivered to a specific tissue or organ and are taken up by cells of that tissue or organ, whereupon the drug having the anticancer activity is released and exhibits anticancer effects, thereby having a prophylactic or therapeutic effect against cancer (e.g., brain tumor, pituitary tumor, glioma, acoustic neurilemoma, retinosarcoma, thyroid cancer, cancer of the larynx, pharyngeal cancer, tongue cancer, thymic cancer, mesothelioma, breast cancer, lung cancer, non-small-cell lung cancer, small-cell lung cancer, stomach cancer, esophageal cancer, duodenal cancer, colorectal cancer, colon cancer, rectal cancer, liver cancer, hepatocarcinoma, pancreatic cancer, pancreatic endocrine tumor, bile duct cancer, gallbladder cancer, cancer of the penis, kidney cancer, renal pelvic cancer, ureteral cancer, renal cell cancer, testicular tumor, prostate cancer, bladder cancer, vulvar cancer, uterine cancer, uterocervical cancer, uterine body cancer, uterine sarcoma, villous diseases, vaginal cancer, ovarian cancer, ovarian germ cell tumor, skin cancer, malignant melanoma, mycosis fungoides, basal cell tumor, soft-tissue sarcoma, malignant lymphoma, Hodgkin's disease, myelodysplastic syndrome, multiple myeloma, leukemia, acute myelogenous leukemia, chronic myelogenous leukemia, acute lymphatic leukemia, chromic lymphatic leukemia, adult T-cell leukemia, chronic myeloproliferative disease, pancreatic endocrine tumor, fibrous histiocytoma, leiomyosarcoma, rhabdomyosarcoma, cancer of unknown primary origin). In the case of sugar chain-modified liposomes, the liposomes are delivered to a specific tissue or organ by the targeting ability of the sugar chains. Even in cases where sugar chains are not attached to the liposomes, because the liposomes of the invention are very stable in the body, the above drugs with an anticancer activity have an increased half-life in the body, enabling the liposomes to effectively reach a specific tissue or organ. The above-mentioned antibodies having an anticancer activity may have either a direct or an indirect anticancer activity.

In cases where the above-mentioned drugs are administered after being included in the sugar chain-modified liposomes of the invention, the drug accumulates at the target site such as cancer tissue as compared with cases where the drug is administered by itself. Compared with administration of the drug alone, at least 2-fold, preferably at least 5-fold, more preferably at least 10-fold, and most preferably at least 50-fold accumulation is possible.

The drug having an anticancer activity may be encapsulated within the liposome or may be bound to the surface of the liposome. When the drug is bound to the liposome surface, proteins such as antibodies may be attached to the surface by the same method as the above-described method for coupling a linker protein, and other compounds may be attached by known methods involving the use of functional groups on those compounds. Encapsulation at the liposome interior is carried out by the following method. Use may be made of a known method to encapsulate a drug or the like in liposomes. For example, drugs are encapsulated in the liposome by using a solution containing the drug and using lipids containing phosphatidylcholines, phosphatidylethanolamines, phosphatidic acids or long-chain alkyl phosphates, gangliosides, glycolipids or phosphatidylglycerols and cholesterols to form the liposomes.

Liposomal preparations obtained by including a drug with an anticancer activity in the liposomes used in the invention thus have an ability to move to various tissues such as cancer tissue that has been selectively controlled, making it possible to boost the potency of therapeutic agents by concentrating them in target cells and tissue or to alleviate side effects by reducing uptake of the drug by other cells or tissues.

The liposomes or sugar chain-modified liposomes of the invention may be administered as drug compositions in various forms. Examples of such dosage forms include the ocular instillation of eye drops, the oral administration of tablets, capsules, pellets, powders and syrups, and the parenteral administration of injections, IV fluids and suppositories. These compositions are manufactured by a known method, and include carriers, diluents and excipients commonly used in the field of pharmaceuticals. For example, gelling agents, lactose, magnesium stearate and the like may be used as carriers and excipients for tablets. Injections are prepared by dissolving, suspending or emulsifying the sugar chain-modified liposomes of the invention in a sterile aqueous or oleaginous liquid commonly used in injections. Aqueous liquids for injection that may be used include physiological saline and isotonic solutions containing glucose and other adjuvants. Concomitant use may also be made of suitable dissolution aids, such as alcohols, polyalcohls such as propylene glycol, and nonionic surfactants. Oleaginous liquids that may be used include sesame oil and soybean oil. Concomitant use may also be made of dissolution aids such as benzyl benzoate and benzyl alcohol.

The route of administration for the drug composition of the invention in mammals includes, but is not limited to, ocular instillation, oral administration, intravenous injection, and intramuscular injection. The dose may be suitably set according to such factors as the severity of the disease, provided the inventive composition is administered to the patient in a pharmacologically effective dose. Here, "administered to the patient in a pharmacologically effective dose" means to administer the drug to the patient at a level suitable for treating the disease. The number of administrations of the drug composition of the invention is selected as appropriate according to the symptoms of the patient.

Liposomes which have sugar chains on the surface are suitable for parenteral administration (intravenous injection) and oral administration. A dose which is from several times to several thousands of times lower than the dose of conventional liposomal preparations will suffice. For example, the amount of drug included in the liposomes per kilogram of body weight may be from 0.0001 to 1,000 mg, preferably from 0.0001 to 10 mg, and more preferably from 0.0001 to 0.1 mg. The liposomes of the invention include also liposomes which do not contain sugar chains having targetability but have hydrophilic compounds attached thereto. In the case of such liposomes as well, hydrophilizing treatment confers a high stability in the body and a long half-life, as a results of which such liposomes have satisfactory effects in low doses.

### EXAMPLES

The present invention is described more fully in the following reference examples, examples and experimental examples. However, these examples should not be construed as limiting the invention.

### Reference Example 1: Preparation of Liposomes

Liposomes were prepared by a previously published technique (Yamazaki, N., Kodama, M and Gabius, H.J. (1994): Methods Enzymol. 242, 56-65) and using modified cholic acid dialysis. That is, 46.9 mg of sodium cholate was added to a combined amount of 45.6 mg of lipids consisting of dipalmitoylphosphatidylcholine, cholesterol, dicetyl phosphate, ganglioside and dipalmitoylphosphatidylethanolamine in the proportions 35:40:5:15:5, and the mixture was dissolved in 3 ml of a chloroform/methanol solution. This solution was evaporated and the precipitate was dried in a vacuum, thereby giving a lipid membrane. The lipid membrane thus obtained was suspended in 3 ml of TAPS buffer (pH 8.4) and sonicated, giving a clear micelle suspension. In addition, the micelle suspension was subjected to ultrafiltration using a PM10 membrane (Amicon Co., USA) and a PBS buffer (pH 7.2), thereby preparing 10 ml of uniform liposomes (average particle size, 100 nm).

### Reference Example 2: Hydrophilizing Treatment on Surface of Liposome Lipid Membrane

Ten milliliters of the liposome solution prepared in Reference Example 1 was subjected to ultrafiltration using an XM300 membrane (Amicon Co., USA) and a CBS buffer (pH 8.5), thereby setting the solution pH to 8.5. Next, ten milliliters of the crosslinking reagent bis(sulfosuccinimidyl) suberate (BS3; Pierce Co., USA) was added and the mixture was stirred at 25°C for 2 hours. This was followed by overnight stirring at 7°C, bringing to completion the chemical bonding reaction between the lipid dipalmitoylphosphatidylethanolamine on the liposome membrane and BS3. This liposome solution was then subjected to ultrafiltration using the XM300 membrane and the CBS buffer (pH 8.5). Next, 40 mg of tris(hydroxymethyl)aminomethane dissolved in CBS buffer (pH 8.5) was added to 10 ml of the liposome solution, and the mixture was stirred at 25°C for two hours, then stirred overnight at 7°C, thereby bringing the chemical bonding reaction between the BS3 bonded to the lipids on the liposome membrane with tris(hydroxymethyl)aminomethane). The hydroxyl groups of tris(hydroxymethyl)aminomethane were thereby attached to the liposome membrane lipid dipalmitoylphosphatidylethanolamine, resulting in hydrophilization by hydration.

### Reference Example 3: Coupling of Human Serum Albumin (HSA) to Liposome Membrane Surface

Coupling was carried out using a coupling reaction in accordance with a previously published technique (Yamazaki, N., Kodama, M. and Gabius, H.J. (1994): Methods Enzymol. 242, 56-65). This reaction was carried out as a two-stage chemical reaction. First, the ganglioside present on the membrane surface of 10 ml of liposomes obtained in Reference Example 2 was added to 43 mg of sodium metaperiodate dissolved in 1 ml of TAPS buffer (pH 8.4), and the mixture was stirred at room temperature for 2 hours to oxidize the periodic acid, following which ultrafiltration using a XM300 membrane and PBS buffer (pH 8.0) was carried out, yielding 10 ml of oxidized liposomes. Twenty milligrams of human serum albumin (HSA) was added to this liposome solution and the mixture was stirred at 25°C for 2 hours, following which 100 µl of 2M NaBH₃CN in PBS (pH 8.0) was added and the mixture was stirred overnight at 10°C, thereby attached HSA to the liposomes by a coupling reaction between the gangliosides on the liposomes and HSA. Next, ultrafiltration was carried out with an XM300 membrane and a CBS buffer (pH 8.5), giving 10 ml of a HSA-coupled liposome solution.

### Reference Example 4: Attaching α-1,2-Mannobiose Disaccharide to Human Serum Albumin (HSA) Coupled to Liposome Membrane Surface

After adding 50 µg of α-1,2-mannobiose disaccharide (Calbiochem Co., USA) to 0.5 ml of an aqueous solution containing 0.25 g of NH₄HCO₃ and stirring at 37°C for three days, the mixture was filtered with a 0.45 µm filter and the amination reaction at the reducing ends of the sugar chains was brought to completion, giving 50 µg of the glycosylamine compound of α-1,2-mannobiose disaccharide. Next, 1 mg of the crosslinking reagent 3,3'-dithiobis(sulfosuccinimidyl propionate) (DTSSP; Pierce Co., USA) was added to a 1 ml portion of the liposome solution obtained in Reference Example 3, following which the mixture was stirred for two hours at 25°C, then stirred overnight at 7°C and subjected to ultrafiltration with an XM300 membrane and CBS buffer (pH 8.5), giving 1 ml of liposomes in which DTSSP is attached to the HSA on the liposomes. Next, 50 µg of the above-mentioned glycosylamine compound of α-1,2-mannobiose disaccharide was added to this liposome solution, the mixture was stirred for two hours at 25°C, then stirred overnight at 7°C and subjected to ultrafiltration with an XM300 membrane and PBS buffer (pH 7.2), carrying out attachment of the α-1,2-mannobiose disaccharide to the DTSSP on the human serum albumin coupled to the membrane surfaces of the liposomes. This procedure yielded 2 ml (total lipid, 2 mg; total protein, 200 µg; average particle size, 100 nm) of the liposomes (abbreviation: A2) which are composed of α-1,2-mannobiose disaccharide, human serum albumin and liposome coupled together as shown in FIG. 1.

### Reference Example 5: Attaching α-1,3-Mannobiose Disaccharide to Human Serum Albumin (HSA) Coupled to Liposome Membrane Surface

After adding 50 µg of α-1,3-mannobiose disaccharide (Calbiochem Co., USA) to 0.5 ml of an aqueous solution containing 0.25 g of NH₄HCO₃ and stirring at 37°C for three days, the mixture was filtered with a 0.45 µm filter and the amination reaction at the reducing ends of the sugar chains was brought to completion, giving 50 µg of the glycosylamine compound of α-1,3-mannobiose disaccharide. Next, 1 mg of the crosslinking reagent 3,3'-dithiobis(sulfosuccinimidyl propionate) (DTSSP; Pierce Co., USA) was added to a 1 ml portion of the liposome solution obtained in Reference Example 3, following which the mixture was stirred for two hours at 25°C, then stirred overnight at 7°C and subjected to ultrafiltration with an XM300 membrane and CBS buffer (pH 8.5), giving 1 ml of liposomes in which DTSSP is attached to the HSA on the liposomes. Next, 50 µg of the above-mentioned glycosylamine compound of α-1,3-mannobiose disaccharide was added to this liposome solution, the mixture was stirred for two hours at 25°C, then stirred overnight at 7°C and subjected to ultrafiltration with an XM300 membrane and PBS buffer (pH 7.2), carrying out attachment of the α-1,3-mannobiose disaccharide to the DTSSP on the human serum albumin coupled to the membrane surfaces of the liposomes. This procedure yielded 2 ml (total lipid, 2 mg; total protein, 200 µg; average particle size, 100 nm) of the liposomes (abbreviation: A3) which are composed of α-1,3-mannobiose disaccharide, human serum albumin and liposome coupled together as shown in FIG. 2.

### Reference Example 6: Attaching α-1,4-Mannobiose Disaccharide to Human Serum Albumin (HSA) Coupled to Liposome Membrane Surface

After adding 50 µg of α-1,4-mannobiose disaccharide (Calbiochem Co., USA) to 0.5 ml of an aqueous solution containing 0.25 g of NH₄HCO₃ and stirring at 37°C for three days, the mixture was filtered with a 0.45 µm filter and the amination reaction at the reducing ends of the sugar chains was brought to completion, giving 50 µg of the glycosylamine compound of α-1,4-mannobiose disaccharide. Next, 1 mg of the crosslinking reagent 3,3'-dithiobis(sulfosuccinimidyl propionate) (DTSSP; Pierce Co., USA) was added to a 1 ml portion of the liposome solution obtained in Reference Example 3, following which the mixture was stirred for two hours at 25°C, then stirred overnight at 7°C and subjected to ultrafiltration with an XM300 membrane and CBS buffer (pH 8.5), giving 1 ml of liposomes in which DTSSP is attached to the HSA on the liposomes. Next, 50 µg of the above-mentioned glycosylamine compound of α-1,4-mannobiose disaccharide was added to this liposome solution, the mixture was stirred for two hours at 25°C, then stirred overnight at 7°C and subjected to ultrafiltration with an XM300 membrane and PBS buffer (pH 7.2), carrying out attachment of the α-1,4-mannobiose disaccharide to the DTSSP on the human serum albumin coupled to the membrane surfaces of the liposomes. This procedure yielded 2 ml ( total lipid, 2 mg; total protein, 200 µg; average particle size, 100 nm)of the liposomes (abbreviation: A4) which are composed of α-1,4-mannobiose disaccharide, human serum albumin and liposome coupled together as shown in FIG. 3.

### Reference Example 7: Attaching α-1,6-Mannobiose Disaccharide to Human Serum Albumin (HSA) Coupled to Liposome Membrane Surface

After adding 50 µg of α-1,6-mannobiose disaccharide (Calbiochem Co., USA) to 0.5 ml of an aqueous solution containing 0.25 g of NH₄HCO₃ and stirring at 37°C for three days, the mixture was filtered with a 0.45 µm filter and the amination reaction at the reducing ends of the sugar chains was brought to completion, giving 50 µg of the glycosylamine compound of α-1,6-mannobiose disaccharide. Next, 1 mg of the crosslinking reagent 3,3'-dithiobis(sulfosuccinimidyl propionate) (DTSSP; Pierce Co., USA) was added to a 1 ml portion of the liposome solution obtained in Reference Example 3, following which the mixture was stirred for two hours at 25°C, then stirred overnight at 7°C and subjected to ultrafiltration with an XM300 membrane and CBS buffer (pH 8.5), giving 1 ml of liposomes in which DTSSP is attached to the HSA on the liposomes. Next, 50 µg of the above-mentioned glycosylamine compound of α-1,6-mannobiose disaccharide was added to this liposome solution, the mixture was stirred for two hours at 25°C, then stirred overnight at 7°C and subjected to ultrafiltration with an XM300 membrane and PBS buffer (pH 7.2), carrying out attachment of the α-1,6-mannobiose disaccharide to the DTSSP on the human serum albumin coupled to the membrane surfaces of the liposomes. This procedure yielded 2 ml (total lipid, 2 mg; total protein, 200 µg; average particle size, 100 nm) of the liposomes (abbreviation: A6) which are composed of α-1,6-mannobiose disaccharide, human serum albumin and liposome coupled together as shown in FIG. 4.

### Reference Example 8: Attaching α-1,3-α-1,6-Mannotriose Trisaccharide to Human Serum Albumin (HSA) Coupled to Liposome Membrane Surface

After adding 50 µg of α-1,3-α-1,6-mannobiose trisaccharide (Calbiochem Co., USA) to 0.5 ml of an aqueous solution containing 0.25 g of NH₄HCO₃ and stirring at 37°C for three days, the mixture was filtered with a 0.45 µm filter and the amination reaction at the reducing ends of the sugar chains was brought to completion, giving 50 µg of the glycosylamine compound of α-1,3-α-1,6-mannobiose trisaccharide. Next, 1 mg of the crosslinking reagent 3,3'-dithiobis(sulfosuccinimidyl propionate) (DTSSP; Pierce Co., USA) was added to a 1 ml portion of the liposome solution obtained in Reference Example 3, following which the mixture was stirred for two hours at 25°C, then stirred overnight at 7°C and subjected to ultrafiltration with an XM300 membrane and CBS buffer (pH 8.5), giving 1 ml of liposomes in which DTSSP is attached to the HSA on the liposomes. Next, 50 µg of the above-mentioned glycosylamine compound of α-1,3-α-1,6-mannobiose trisaccharide was added to this liposome solution, the mixture was stirred for two hours at 25°C, then stirred overnight at 7°C and subjected to ultrafiltration with an XM300 membrane and PBS buffer (pH 7.2), carrying out attachment of the α-1,3-α-1,6-mannobiose trisaccharide to the DTSSP on the human serum albumin coupled to the membrane surfaces of the liposomes. This procedure yielded 2 ml (total lipid, 2 mg; total protein, 200 µg; average particle size, 100 nm) of the liposomes (abbreviation: A36) which are composed of α-1,3-α-1,6-mannobiose trisaccharide, human serum albumin and liposome coupled together as shown in FIG. 5.

### Reference Example 9: Attaching Oligomannose-3 Pentasaccharide to Human Serum Albumin (HSA) Coupled to Liposome Membrane Surface

After adding 50 µg of oligomannose-3 pentasaccharide (Calbiochem Co., USA) to 0.5 ml of an aqueous solution containing 0.25 g of NH₄HCO₃ and stirring at 37°C for three days, the mixture was filtered with a 0.45 µm filter and the amination reaction at the reducing ends of the sugar chains was brought to completion, giving 50 µg of the glycosylamine compound of oligomannose-3 pentasaccharide. Next, 1 mg of the crosslinking reagent 3,3'-dithiobis(sulfosuccinimidyl propionate) (DTSSP; Pierce Co., USA) was added to a 1 ml portion of the liposome solution obtained in Reference Example 3, following which the mixture was stirred for two hours at 25°C, then stirred overnight at 7°C and subjected to ultrafiltration with an XM300 membrane and CBS buffer (pH 8.5), giving 1 ml of liposomes in which DTSSP is attached to the HSA on the liposomes. Next, 50 µg of the above-mentioned glycosylamine compound of oligomannose-3 pentasaccharide was added to this liposome solution, the mixture was stirred for two hours at 25°C, then stirred overnight at 7°C and subjected to ultrafiltration with an XM300 membrane and PBS buffer (pH 7.2), carrying out attachment of the oligomannose-3 pentasaccharide to the DTSSP on the human serum albumin coupled to the membrane surfaces of the liposomes. This procedure yielded 2 ml (total lipid, 2 mg; total protein, 200 µg; average particle size, 100 nm) of the liposomes (abbreviation: Man3) which are composed of oligomannose-3 pentasaccharide, human serum albumin and liposome coupled together as shown in FIG. 6.

### Reference Example 10: Attaching Oligomannose-4b Hexasaccharide to Human Serum Albumin (HSA) Coupled to Liposome Membrane Surface

After adding 50 µg of oligomannose-4b hexasaccharide (Calbiochem Co., USA) to 0.5 ml of an aqueous solution containing 0.25 g of NH₄HCO₃ and stirring at 37°C for three days, the mixture was filtered with a 0.45 µm filter and the amination reaction at the reducing ends of the sugar chains was brought to completion, giving 50 µg of the glycosylamine compound of oligomannose-4b hexasaccharide. Next, 1 mg of the crosslinking reagent 3,3'-dithiobis(sulfosuccinimidyl propionate) (DTSSP; Pierce Co., USA) was added to a 1 ml portion of the liposome solution obtained in Reference Example 3, following which the mixture was stirred for two hours at 25°C, then stirred overnight at 7°C and subjected to ultrafiltration with an XM300 membrane and CBS buffer (pH 8.5), giving 1 ml of liposomes in which DTSSP is attached to the HSA on the liposomes. Next, 50 µg of the above-mentioned glycosylamine compound of oligomannose-4b hexasaccharide was added to this liposome solution, the mixture was stirred for two hours at 25°C, then stirred overnight at 7°C and subjected to ultrafiltration with an XM300 membrane and PBS buffer (pH 7.2), carrying out attachment of the oligomannose-4b hexasaccharide to the DTSSP on the human serum albumin coupled to the membrane surfaces of the liposomes. This procedure yielded 2 ml (total lipid, 2 mg; total protein, 200 µg; average particle size, 100 nm) of the liposomes (abbreviation: Man4b) which are composed of oligomannose-4b hexasaccharide, human serum albumin and liposome coupled together as shown in FIG. 7.

### Reference Example 11: Attaching Oligomannose-5 Heptasaccharide to Human Serum Albumin (HSA) Coupled to Liposome Membrane Surface

After adding 50 µg of oligomannose-5 heptasaccharide (Calbiochem Co., USA) to 0.5 ml of an aqueous solution containing 0.25 g of NH₄HCO₃ and stirring at 37°C for three days, the mixture was filtered with a 0.45 µm filter and the amination reaction at the reducing ends of the sugar chains was brought to completion, giving 50 µg of the glycosylamine compound of oligomannose-5 heptasaccharide. Next, 1 mg of the crosslinking reagent 3,3'-dithiobis(sulfosuccinimidyl propionate) (DTSSP; Pierce Co., USA) was added to a 1 ml portion of the liposome solution obtained in Reference Example 3, following which the mixture was stirred for two hours at 25°C, then stirred overnight at 7°C and subjected to ultrafiltration with an XM300 membrane and CBS buffer (pH 8.5), giving 1 ml of liposomes in which DTSSP is attached to the HSA on the liposomes. Next, 50 µg of the above-mentioned glycosylamine compound of oligomannose-5 heptasaccharide was added to this liposome solution, the mixture was stirred for two hours at 25°C, then stirred overnight at 7°C and subjected to ultrafiltration with an XM300 membrane and PBS buffer (pH 7.2), carrying out attachment of the oligomannose-5 heptasaccharide to the DTSSP on the human serum albumin coupled to the membrane surfaces of the liposomes. This procedure yielded 2 ml(total lipid, 2 mg; total protein, 200 µg; average particle size, 100 nm) of the liposomes (abbreviation: Man5) which are composed of oligomannose-5 heptasaccharide, human serum albumin and liposome coupled together as shown in FIG. 8.

### Reference Example 12: Attaching Oligomannose-6 Octasaccharide to Human Serum Albumin (HSA) Coupled to Liposome Membrane Surface

After adding 50 µg of oligomannose-6 octasaccharide (Calbiochem Co., USA) to 0.5 ml of an aqueous solution containing 0.25 g of NH₄HCO₃ and stirring at 37°C for three days, the mixture was filtered with a 0.45 µm filter and the amination reaction at the reducing ends of the sugar chains was brought to completion, giving 50 µg of the glycosylamine compound of oligomannose-6 octasaccharide. Next, 1 mg of the crosslinking reagent 3,3'-dithiobis(sulfosuccinimidyl propionate) (DTSSP; Pierce Co., USA) was added to a 1 ml portion of the liposome solution obtained in Reference Example 3, following which the mixture was stirred for two hours at 25°C, then stirred overnight at 7°C and subjected to ultrafiltration with an XM300 membrane and CBS buffer (pH 8.5), giving 1 ml of liposomes in which DTSSP is attached to the HSA on the liposomes. Next, 50 µg of the above-mentioned glycosylamine compound of oligomannose-6 octasaccharide was added to this liposome solution, the mixture was stirred for two hours at 25°C, then stirred overnight at 7°C and subjected to ultrafiltration with an XM300 membrane and PBS buffer (pH 7.2), carrying out attachment of the oligomannose-6 octasaccharide to the DTSSP on the human serum albumin coupled to the membrane surfaces of the liposomes. This procedure yielded 2 ml (total lipid, 2 mg; total protein, 200 µg; average particle size, 100 nm) of the liposomes (abbreviation: Man6) which are composed of oligomannose-6 octasaccharide, human serum albumin and liposome coupled together as shown in FIG. 9.

### Reference Example 13: Attaching Oligomannose-7 Nonasaccharide to Human Serum Albumin (HSA) Coupled to Liposome Membrane Surface

After adding 50 µg of oligomannose-7 nonasaccharide (Calbiochem Co., USA) to 0.5 ml of an aqueous solution containing 0.25 g of NH₄HCO₃ and stirring at 37°C for three days, the mixture was filtered with a 0.45 µm filter and the amination reaction at the reducing ends of the sugar chains was brought to completion, giving 50 µg of the glycosylamine compound of oligomannose-7 nonasaccharide. Next, 1 mg of the crosslinking reagent 3,3'-dithiobis(sulfosuccinimidyl propionate) (DTSSP; Pierce Co., USA) was added to a 1 ml portion of the liposome solution obtained in Reference Example 3, following which the mixture was stirred for two hours at 25°C, then stirred overnight at 7°C and subjected to ultrafiltration with an XM300 membrane and CBS buffer (pH 8.5), giving 1 ml of liposomes in which DTSSP is attached to the HSA on the liposomes. Next, 50 µg of the above-mentioned glycosylamine compound of oligomannose-7 nonasaccharide was added to this liposome solution, the mixture was stirred for two hours at 25°C, then stirred overnight at 7°C and subjected to ultrafiltration with an XM300 membrane and PBS buffer (pH 7.2), carrying out attachment of the oligomannose-7 nonasaccharide to the DTSSP on the human serum albumin coupled to the membrane surfaces of the liposomes. This procedure yielded 2 ml (total lipid, 2 mg; total protein, 200 µg; average particle size, 100 nm) of the liposomes (abbreviation: Man7) which are composed of oligomannose-7 nonasaccharide, human serum albumin and liposome coupled together as shown in FIG. 10.

### Reference Example 14: Attaching Oligomannose-8 Decasaccharide to Human Serum Albumin (HSA) Coupled to Liposome Membrane Surface

After adding 50 µg of oligomannose-8 decasaccharide (Calbiochem Co., USA) to 0.5 ml of an aqueous solution containing 0.25 g of NH₄HCO₃ and stirring at 37°C for three days, the mixture was filtered with a 0.45 µm filter and the amination reaction at the reducing ends of the sugar chains was brought to completion, giving 50 µg of the glycosylamine compound of oligomannose-8 decasaccharide. Next, 1 mg of the crosslinking reagent 3,3'-dithiobis(sulfosuccinimidyl propionate) (DTSSP; Pierce Co., USA) was added to a 1 ml portion of the liposome solution obtained in Reference Example 3, following which the mixture was stirred for two hours at 25°C, then stirred overnight at 7°C and subjected to ultrafiltration with an XM300 membrane and CBS buffer (pH 8.5), giving 1 ml of liposomes in which DTSSP is attached to the HSA on the liposomes. Next, 50 µg of the above-mentioned glycosylamine compound of oligomannose-8 decasaccharide was added to this liposome solution, the mixture was stirred for two hours at 25°C, then stirred overnight at 7°C and subjected to ultrafiltration with an XM300 membrane and PBS buffer (pH 7.2), carrying out attachment of the oligomannose-8 decasaccharide to the DTSSP on the human serum albumin coupled to the membrane surfaces of the liposomes. This procedure yielded 2 ml (total lipid, 2 mg; total protein, 200 µg; average particle size, 100 nm) of the liposomes (abbreviation: Man8) which are composed of oligomannose-8 decasaccharide, human serum albumin and liposome coupled together as shown in FIG. 11.

### Reference Example 15: Attaching Oligomannose-9 Undecasaccharide to Human Serum Albumin (HSA) Coupled to Liposome Membrane Surface

After adding 50 µg of oligomannose-9 undecasaccharide (Calbiochem Co., USA) to 0.5 ml of an aqueous solution containing 0.25 g of NH₄HCO₃ and stirring at 37°C for three days, the mixture was filtered with a 0.45 µm filter and the amination reaction at the reducing ends of the sugar chains was brought to completion, giving 50 µg of the glycosylamine compound of oligomannose-9 undecasaccharide. Next, 1 mg of the crosslinking reagent 3,3'-dithiobis(sulfosuccinimidyl propionate) (DTSSP; Pierce Co., USA) was added to a 1 ml portion of the liposome solution obtained in Reference Example 3, following which the mixture was stirred for two hours at 25°C, then stirred overnight at 7°C and subjected to ultrafiltration with an XM300 membrane and CBS buffer (pH 8.5), giving 1 ml of liposomes in which DTSSP is attached to the HSA on the liposomes. Next, 50 µg of the above-mentioned glycosylamine compound of oligomannose-9 undecasaccharide was added to this liposome solution, the mixture was stirred for two hours at 25°C, then stirred overnight at 7°C and subjected to ultrafiltration with an XM300 membrane and PBS buffer (pH 7.2), carrying out attachment of the oligomannose-9 undecasaccharide to the DTSSP on the human serum albumin coupled to the membrane surfaces of the liposomes. This procedure yielded 2 ml (total lipid, 2 mg; total protein, 200 µg; average particle size, 100 nm) of the liposomes (abbreviation: Man9) which are composed of oligomannose-9 undecasaccharide, human serum albumin and liposome coupled together as shown in FIG. 12.

### Reference Example 16: Attaching Tris(hydroxymethyl)aminomethane to Human Serum Albumin (HSA) Coupled to Liposome Membrane Surface

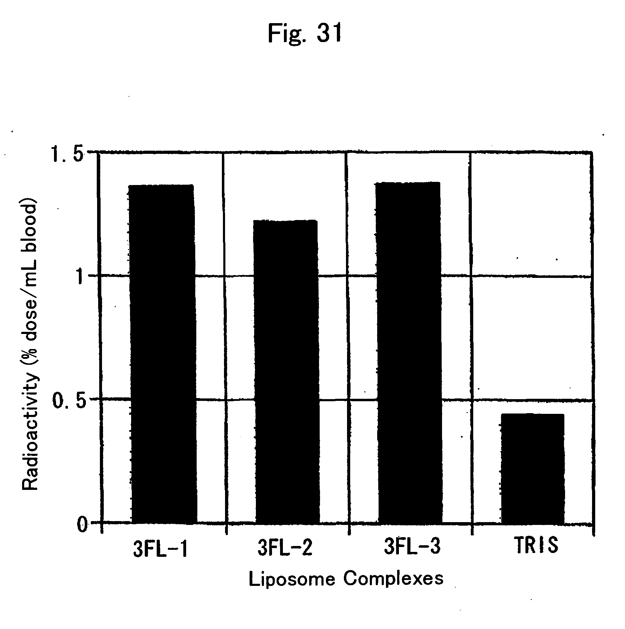

To prepare liposomes for use as a control, 1 mg of the crosslinking reagent 3,3'-dithiobis(sulfosuccinimidyl propionate) (DTSSP; Pierce Co., USA) was added to a 1 ml portion of the liposome solution obtained in Reference Example 3, following which the mixture was stirred for two hours at 25°C, then stirred overnight at 7°C and subjected to ultrafiltration with an XM300 membrane and CBS buffer (pH 8.5), giving 1 ml of liposomes in which DTSSP is attached to the HSA on the liposomes. Next, 13 mg of tris(hydroxymethyl)aminomethane (Wako Co., Japan) was added to this liposome solution, the mixture was stirred for two hours at 25°C then stirred overnight at 7°C and subjected to ultrafiltration with an XM300 membrane and PBS buffer (pH 7.2), carrying out attachment of the tris(hydroxymethyl)aminomethane to the DTSSP on the human serum albumin coupled to the membrane surfaces of the liposomes. This procedure yielded 2 ml (total lipid, 2 mg; total protein, 200 µg; average particle size, 100 nm) of liposomes (abbreviation: TRIS) coupled with tris(hydroxymethyl)aminomethane, DTSSP on the human serum albumin and the liposomes for use as a control as shown in FIG. 31.

### Reference Example 17: Hydrophilization of Human Serum Albumin (HSA) Coupled to Liposome Membrane Surface

The liposomes having attached thereto one of 12 types of sugar chains that were prepared in Reference Examples 4 to 15 were each separately subjected to hydrophilizing treatment of the HSA protein surface on the liposomes by the following procedure. Tris(hydroxymethyl)aminomethane, 13 mg, was added to 2 ml of each of the twelve types of sugar chain-coupled liposomes, following which the respective mixtures were stirred for two hours at 25°C, then stirred overnight at 7°C and subjected to ultrafiltration with an XM300 membrane and PBS buffer (pH 7.2). This procedure yielded as the final products 2 ml of each of the twelve types of sugar chain-coupled liposome complexes that had been hydration treated (abbreviations: A2, A3, A4, A6, A36, Man3, Man4, Man5, Man6, Man7, Man8, Man9).

### Reference Example 18: Measurement of Lectin Binding Activity-Inhibiting Effects of Various Sugar Chain-Coupled Liposome Complexes

The in vitro lectin binding activities of the 12 types of sugar chain-coupled liposome complexes prepared in Reference Examples 4 to 15 and Reference Example 17 were measured by inhibition tests using lectin-immobilized microplates, in accordance with a conventional method (Yamazaki, N. (1999): Drug Delivery System 14, 498-505). That is, lectin (Con A; R&D Systems Co., USA) was immobilized on a 96-well microplate. To this lectin-immobilized plate were added 0.1 µg of biotinated fetuin as control ligands and different concentrations of the various sugar chain-coupled liposome complexes (representing protein weights of 0.01 µg, 0.04 µg, 0.11 µg, 0.33 µg or 1 µg), followed by 2 hours of incubation at 4°C. The plate was then washed three times with PBS (pH 7.2), after which horseradish peroxidase (HRPO)-coupled streptavidin was added and the plate was incubated for 1 hour at 4°C, then washed three times with PBS (pH 7.2). Next, peroxidase substrate was added, the plate was held stationary at room temperature, and the absorbance at 405 nm was measured with a microplate reader (Molecular Devices Corp., USA). The biotinization of fetuin was carried out by sulfo-NHS-biotin reagent (Pierce Co., USA) treatment, followed by purification with a Centricon-30 (Amicon Co., USA). The HRPO-coupled streptavidin was prepared by HRPO oxidation and by the coupling of streptavidin through reductive amination using NaBH₃CN. The measurement results are shown in Table 1.

### [Table 1]

### Targeted Liposomes

**Table 1: Experimental results showing the lectin binding inhibition effects by various sugar chain-coupled liposome complexes.**

| Liposome complex | Inhibitory effects (absorbance) of liposome complexes at various concentrations (µg of protein) | | | | |
|---|---|---|---|---|---|
| | 0.006 µg | 0.02 µg | 0.06 µg | 0.17 µg | 0.5 µg |
| A2 | 0.192 | 0.196 | 0.192 | 0.169 | 0.155 |
| A3 | 0.178 | 0.178 | 0.178 | 0.170 | 0.142 |
| A4 | 0.192 | 0.196 | 0.192 | 0.175 | 0.153 |
| A6 | 0.182 | 0.196 | 0.182 | 0.169 | 0.151 |
| A36 | 0.205 | 0.215 | 0.205 | 0.192 | 0.150 |
| Man3 | 0.201 | 0.211 | 0.201 | 0.177 | 0.144 |
| Man4 | 0.171 | 0.203 | 0.171 | 0.157 | 0.148 |
| Man5 | 0.215 | 0.221 | 0.215 | 0.196 | 0.164 |
| Man6 | 0.210 | 0.222 | 0.210 | 0.207 | 0.125 |
| Man7 | 0.213 | 0.214 | 0.213 | 0.183 | 0.137 |
| Man8 | 0.211 | 0.216 | 0.211 | 0.188 | 0.132 |
| Man9 | 0.208 | 0.211 | 0.208 | 0.186 | 0.135 |

### Reference Example 19: ¹²⁵I Labeling of Various Sugar Chain-Coupled Liposomes by Chloramine-T Method

A chloramine-T (Wako Pure Chemical Co., Japan) solution and a sodium disulfite solution were prepared at the time of use to respective concentrations of 3 mg/ml and 5 mg/ml. The 12 types of sugar chain-coupled liposomes and the tris(hydroxymethyl)aminomethane-coupled liposomes prepared in Reference Examples 4 to 16 were placed in amounts of 50 µl each in separate Eppendorf tubes, following which 15 µl of ¹²⁵I-NaI (NEN Life Science Product, Inc., USA) and 10 µl of the chloramine-T solution were added and reacted. Ten microliters of the chloramine-T solution was added at 5-minute intervals. Fifteen minutes after repeating this operation twice, 100 µl of the sodium disulfite solution was added as a reducing agent, bringing the reaction to a halt. Next, the tube contents were applied to Sephadex G-50 (Pharmacia Biotech, Sweden) column chromatography and eluted with PBS, thereby purifying the labeled bodies. Finally, in each case, unlabeled liposome complex was added to adjust the specific activity to 4×10⁶ Bq/mg protein, thereby giving 13 types of ¹²⁵I-labeled liposome solutions.

### Reference Example 20: Measurement of Distributions of Each Type of Sugar Chain-Coupled Liposome Complex to Various Tissues in Tumor-Bearing Mice

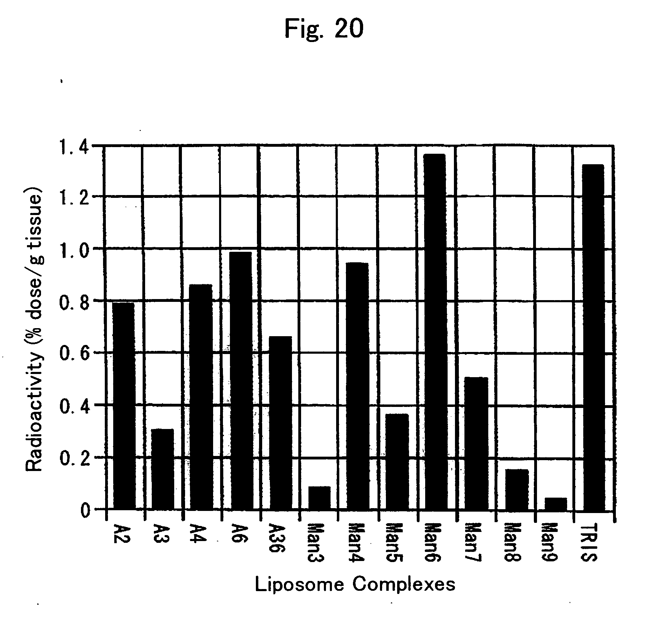

Ehrlich ascites tumor (EAT) cells (approx. 2×10⁷) were implanted subcutaneously in the femurs of female ddY mice (7-week old). Animals in which the cancer tissue grew to from 0.3 to 0.6 g (after 6 to 8 days) were used in this experiment. These tumor-bearing mice were administered, by injection in the caudal vein, 0.2 ml (representing 3 µg of protein per animal) of one of the 12 types of sugar chain-coupled or tris(hydroxymethyl)aminomethane-coupled liposome complexes that were ¹²⁵I-labeled in Reference Example 19. Sixty minutes later, certain tissues (blood, liver, spleen, lungs, brain, cancer tissue, inflamed tissue peripheral to cancer, and lymph nodes) were removed, and the radioactivity of each tissue was measured with a gamma counter (Aloka ARC 300). The distribution of radioactivity to the various tissues was represented as the proportion of radioactivity per gram of each type of tissue relative to the total radioactivity administered (% of dose/g of tissue). The results are shown in FIGS. 13 to 22.

### Reference Example 21: Attaching 3'-Sialyllactose Trisaccharide to Human Serum Albumin (HSA) Coupled to Liposome Membrane Surface (3 types having different sugar chain bonding amounts)

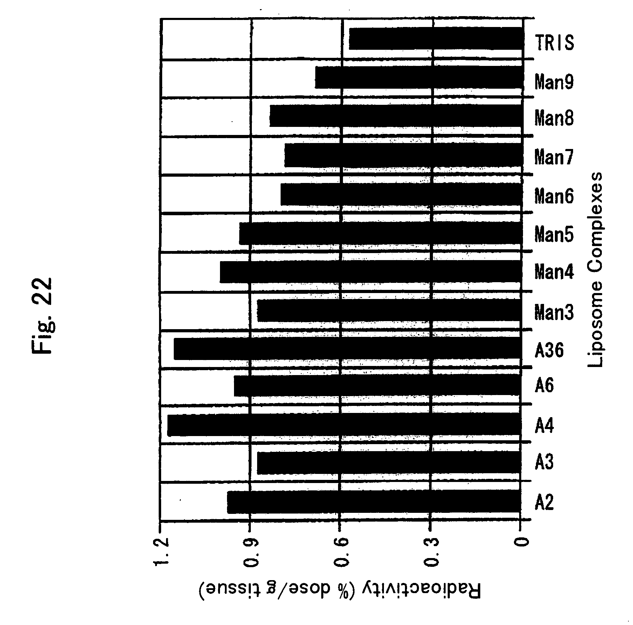

3'-Sialyllactose trisaccharide (Wako Pure Chemical Co., Japan) was added in an amount of (1) 50 µg, (2) 200 µg or (3) 1 mg to 0.5 ml of an aqueous solution containing 0.25 g of NH₄HCO₃, following which the mixture was stirred at 37°C for three days, then filtered with a 0.45 µm filter and the amination reaction at the reducing ends of the sugar chains was brought to completion, giving 50 µg of a glycosylamine compound of 3'-sialyllactose trisaccharide. Next, 1 mg of the crosslinking reagent 3,3'-dithiobis(sulfosuccinimidyl propionate) (DTSSP; Pierce Co., USA) was added to a 1 ml portion of the liposome solution obtained in Reference Example 3, following which the mixture was stirred for two hours at 25°C, then stirred overnight at 7°C and subjected to ultrafiltration with an XM300 membrane and CBS buffer (pH 8.5), giving 1 ml of liposomes in which DTSSP is attached to the HSA on the liposomes. Next, 50 µg of the above-mentioned glycosylamine compound of 3'-sialyllactose trisaccharide was added to this liposome solution, the mixture was stirred for two hours at 25°C, then stirred overnight at 7°C and subjected to ultrafiltration with an XM300 membrane and PBS buffer (pH 7.2), carrying out attachment of the 3'-sialyllactose trisaccharide to the DTSSP on the human serum albumin coupled to the membrane surfaces of the liposomes. This procedure yielded 2 ml each (total lipid, 2 mg; total protein, 200 µg; average particle size, 100 nm) of the three types of liposomes (abbreviation: 1) 3SL-1, 2) 3SL-2, 3) 3SL-3) which are composed of 3'-sialyllactose trisaccharide, human serum albumin and liposome coupled together as shown in FIG. 22, and have differing sugar chain bonding amounts.

### Reference Example 22: Attaching 6'-Sialyllactose Trisaccharide to Human Serum Albumin (HSA) Coupled to Liposome Membrane Surface (3 types having different sugar chain bounding amounts)

6'-Sialyllactose trisaccharide (Wako Pure Chemical Co., Japan) was added in an amount of (1) 50 µg, (2) 200 µg or (3) 1 mg to 0.5 ml of an aqueous solution containing 0.25 g of NH₄HCO₃, following which the mixture was stirred at 37°C for three days, then filtered with a 0.45 µm filter and the amination reaction at the reducing ends of the sugar chains was brought to completion, giving 50 µg of a glycosylamine compound of 6'-sialyllactose trisaccharide. Next, 1 mg of the crosslinking reagent 3,3'-dithiobis(sulfosuccinimidyl propionate) (DTSSP; Pierce Co., USA) was added to a 1 ml portion of the liposome solution obtained in Reference Example 3, following which the mixture was stirred for two hours at 25°C, then stirred overnight at 7°C and subjected to ultrafiltration with an XM300 membrane and CBS buffer (pH 8.5), giving 1 ml of liposomes in which DTSSP is attached to the HSA on the liposomes. Next, 50 µg of the above-mentioned glycosylamine compound of 6'-sialyllactose trisaccharide was added to this liposome solution, the mixture was stirred for two hours at 25°C, then stirred overnight at 7°C and subjected to ultrafiltration with an XM300 membrane and PBS buffer (pH 7.2), carrying out attachment of the 6'-sialyllactose trisaccharide to the DTSSP on the human serum albumin coupled to the membrane surfaces of the liposomes. This procedure yielded 2 ml each (total lipid, 2 mg; total protein, 200 µg; average particle size, 100 nm) of the three types of liposomes (abbreviation: 1) 6SL-1, 2) 6SL-2, 3) 6SL-3) which are composed of 6'-sialyllactose trisaccharide, human serum albumin and liposome coupled together as shown in FIG. 23 and have differing sugar chain bonding amounts.

### Reference Example 23: Attaching 3'-Sialyllactosamine Saccharide to Human Serum Albumin (HSA) Coupled to Liposome Membrane Surface (3 types having different sugar chain bonding amounts)

3'-Sialyllactosamine saccharide (Wako Pure Chemical Co., Japan) was added in an amount of (1) 50 µg, (2) 200 µg or (3) 1 mg to 0.5 ml of an aqueous solution containing 0.25 g of NH₄HCO₃, following which the mixture was stirred at 37°C for three days, then filtered with a 0.45 µm filter and the amination reaction at the reducing ends of the sugar chains was brought to completion, giving 50 µg of a glycosylamine compound of 3'-sialyllactosamine saccharide. Next, 1 mg of the crosslinking reagent 3,3'-dithiobis(sulfosuccinimidyl) propionate (DTSSP; Pierce Co., USA) was added to a 1 ml portion of the liposome solution obtained in Reference Example 3, following which the mixture was stirred for two hours at 25°C, then stirred overnight at 7°C and subjected to ultrafiltration with an XM300 membrane and CBS buffer (pH 8.5), giving 1 ml of liposomes in which DTSSP is attached to the HSA on the liposomes. Next, 50 µg of the above-mentioned glycosylamine compound of 3'-sialyllactosamine saccharide was added to this liposome solution, the mixture was stirred for two hours at 25°C, then stirred overnight at 7°C and subjected to ultrafiltration with an XM300 membrane and PBS buffer (pH 7.2), carrying out attachment of the 3'-sialyllactosamine saccharide to the DTSSP on the human serum albumin coupled to the membrane surfaces of the liposomes. This procedure yielded 2 ml each (total lipid, 2 mg; total protein, 200 µg; average particle size, 100 nm) of the three types of liposomes (abbreviation: 1) 3SLN-1, 2) 3SLN-2, 3) 3SLN-3; which are composed of 3'-sialyllactosamine saccharide, human serum albumin and liposome coupled together as shown in FIG. 24, and have differing sugar chain bonding amounts.

### Reference Example 24: Attaching 6'-Sialyllactosamine Saccharide to Human Serum Albumin (HSA) Coupled to Liposome Membrane Surfaces (3 types having different sugar chain bonding amounts)

6'-Sialyllactosamine saccharide (Wako Pure Chemical Co., Japan) was added in an amount of (1) 50 µg, (2) 200 µg or (3) 1 mg to 0.5 ml of an aqueous solution containing 0.25 g of NH₄HCO₃, following which the mixture was stirred at 37°C for three days, then filtered with a 0.45 µm filter and the amination reaction at the reducing ends of the sugar chains was brought to completion, giving 50 µg of a glycosylamine compound of 6'-sialyllactosamine saccharide. Next, 1 mg of the crosslinking reagent 3,3'-dithiobis(sulfosuccinimidyl) propionate (DTSSP; Pierce Co., USA) was added to a 1 ml portion of the liposome solution obtained in Reference Example 3, following which the mixture was stirred for two hours at 25°C, then stirred overnight at 7°C and subjected to ultrafiltration with an XM300 membrane and CBS buffer (pH 8.5), giving 1 ml of liposomes in which DTSSP is attached to the HSA on the liposomes. Next, 50 µg of the above-mentioned glycosylamine compound of 6'-sialyllactosamine saccharide was added to this liposome solution, the mixture was stirred for two hours at 25°C, then stirred overnight at 7°C and subjected to ultrafiltration with an XM300 membrane and PBS buffer (pH 7.2), carrying out attachment of the 6'-sialyllactosamine saccharide to the DTSSP on the human serum albumin coupled to the membrane surfaces of the liposomes. This procedure yielded 2 ml each (total lipid, 2 mg; total protein, 200 µg; average particle size, 100 nm) of the three types of liposomes (abbreviation: 1) 6SLN-1, 2) 6SLN-2, 3) 6SLN-3; which are composed of 6'-sialyllactosamine saccharide, human serum albumin and liposome coupled together as shown in FIG. 25, and have differing sugar chain bonding amounts.

### Reference Example 25: Hydrophilization of Human Serum Albumin (HSA) Coupled to Liposome Membrane Surface

The liposomes having attached thereto one of 12 types of sugar chains that were prepared in Reference Examples 21 to 24 were each separately subjected to hydrophilizing treatment of the HSA protein surface on the liposomes by the following procedure. Tris(hydroxymethyl)aminomethane, 13 mg, was added to 2 ml of each of the twelve types of sugar chain-coupled liposomes, following which the respective mixtures were stirred for two hours at 25°C, then stirred overnight at 7°C and subjected to ultrafiltration with an XM300 membrane and PBS buffer (pH 7.2). This procedure yielded as the final products 2 ml of each (total lipid, 2 mg; total protein, 200 µg; average particle size, 100 nm) of the 12 types of sugar chain-coupled liposome complexes that had been hydrophilization treated (abbreviations: 3SL-2, 3SL-2, 3SL-3, 6SL-1, 6SL-2, 6SL-3, 3SLN-1, 3SLN-2, 3SLN-3, 6SLN-1, 6SLN-2, 6SLN-3;).

### Reference Example 26: Measurement of Lectin Binding Activity-Inhibiting Effect of Various Sugar Chain-Coupled Liposome Complexes

The in vitro lectin binding activities of the 12 types of sugar chain-coupled liposome complexes prepared by the procedures described in Reference Examples 21 to 24 and Reference Example 16 were measured by inhibition tests using lectin-immobilized microplates, in accordance with a conventional method (Yamazaki, N. (1999): Drug Delivery System 14, 498-505). That is, lectin (E-selectin; R&D Systems Co., USA) was immobilized on a 96-well microplate. To this lectin-immobilized plate were added 0.1 µg of biotinated fucosylated fetuin as control ligands and different concentrations of the various sugar chain-coupled liposome complexes (representing protein weights of 0.01 µg, 0.04 µg, 0.11 µg, 0.33 µg or 1 µg), followed by 2 hours of incubation at 4°C. The plate was then washed three times with PBS (pH 7.2), after which horseradish peroxidase (HRPO)-coupled streptavidin was added and the plate was incubated for 1 hour at 4°C, then washed three times with PBS (pH 7.2). Next, peroxidase substrate was added, the plate was held stationary at room temperature, and the absorbance at 405 nm was measured with a microplate reader (Molecular Devices Corp., USA). The biotinization of fucosylated fetuin was carried out by sulfo-NHS-biotin reagent (Pierce Co., USA) treatment, followed by purification with a Centricon-30 (Amicon Co., USA). The HRPO-coupled streptavidin was prepared by HRPO oxidation and by the coupling of streptavidin through reductive amination using NaBH₃CN. The measurement results are shown in Table 2.

### [Table 2]

### Intestinal Absorption-Controlled Liposomes

**Table 2**

| Liposome complex | Inhibitory effects (absorbance) of liposome complexes at various concentrations (µg of protein) | | | | |
|---|---|---|---|---|---|
| | 0.01 µg | 0.04 µg | 0.11 µg | 0.33 µg | 1 µg |
| 3SL-1 | 0.154 | 0.147 | 0.135 | 0.120 | 0.097 |
| 3SL-2 | 0.149 | 0.142 | 0.124 | 0.118 | 0.098 |
| 3SL-3 | 0.214 | 0.214 | 0.210 | 0.183 | 0.167 |
| 6SL-1 | 0.177 | 0.171 | 0.167 | 0.160 | 0.114 |
| 6SL-2 | 0.196 | 0.184 | 0.169 | 0.160 | 0.159 |
| 6SL-3 | 0.214 | 0.207 | 0.196 | 0.192 | 0.183 |
| 3SLN-1 | 0.219 | 0.198 | 0.180 | 0.164 | 0.119 |
| 3SLN-2 | 0.155 | 0.155 | 0.151 | 0.119 | 0.096 |
| 3 SLN-3 | 0.216 | 0.198 | 0.187 | 0.146 | 0.132 |
| 6SLN-1 | 0.257 | 0.246 | 0.233 | 0.200 | 0.151 |
| 6SLN-2 | 0.250 | 0.250 | 0.230 | 0.199 | 0.158 |
| 6SLN-3 | 0.248 | 0.231 | 0.227 | 0.201 | 0.144 |

### Reference Example 27: ¹²⁵I Labeling of Various Sugar Chain-Coupled Liposomes by Chloramine-T Method

A chloramine-T (Wako Pure Chemical Co., Japan) solution and a sodium disulfite solution were prepared at the time of use to respective concentrations of 3 mg/ml and 5 mg/ml. The 13 types of sugar chain-coupled liposomes and the tris(hydroxymethyl)aminomethane-coupled liposomes prepared in Reference Examples 21 to 24 and Reference Example 16 were placed in amounts of 50 µl each in separate Eppendorf tubes, following which 15 µl of ¹²⁵I-NaI (NEN Life Science Product, Inc., USA) and 10 µl of chloramine-T solution were added and reacted. Ten microliters of the chloramine-T solution was added at 5-minute intervals. Fifteen minutes after repeating this operation twice, 100 µl of the sodium disulfite solution was added as a reducing agent, bringing reaction to a halt. Next, the tube contents were applied to Sephadex G-50 (Pharmacia Biotech, Sweden) column chromatography and eluted with PBS, thereby purifying the labeled bodies. Finally, in each case, unlabeled liposome complex was added to adjust the specific activity to 4×10⁶ Bq/mg protein, thereby giving 13 types of ¹²⁵I-labeled liposome solutions.

### Reference Example 28: Measurement of Transition of Each Type of Sugar Chain-Coupled Liposome Complex from Intestinal Tract to Blood in Mice

Female ddY mice (7-week old) that had been deprived of food (other than water) for one full day were forcibly administered to the intestinal tract, using an oral probe for mice, 0.2 ml of one of the 13 types of sugar chain-coupled or tris(hydroxymethyl)aminomethane-coupled liposome complexes that were ¹²⁵I-labeled in Reference Example 27 in respective amounts of 3 µg of the protein per animal. Ten minutes later, with the animals under nembutal anesthesia, 1 ml of blood was collected from the descending aorta, and the ¹²⁵I radioactivity in the blood was measured with a gamma counter (Alola ARC 300). In addition, to investigate the stability in vivo of each type of liposome complex, the serum from each blood sample was again subjected to chromatography with Sephadex G-50. In each case, most of the radioactivity was observed in the high-molecular-weight void fractions, demonstrating that each type of liposome complex was stable in vivo as well. The transition rate of the radioactivity from the intestinal tract to the blood was represented as the proportion of radioactivity per ml of blood relative to the total radioactivity administered (% of dose/ml of blood). The results are shown in FIGS. 27 to 31.

### Example 1

### (1) Preparation of Docetaxel Hydrate-Encapsulating Liposomes

Docetaxel hydrate-encapsulated liposomes were prepared using cholic acid dialysis. That is, dipalmitoylphosphatidylcholine, cholesterol, dicetyl phosphate, ganglioside and dipalmitoylphosphatidylethanolamine were mixed in the proportions 35:40:5:15:5 so that the combined amount of lipids was 45.6 mg, after which 46.9 mg of sodium cholate was added and the resulting mixture was dissolved in 3 ml of a chloroform/methanol solution. This solution was evaporated and the precipitate was dried in a vacuum, thereby giving a lipid membrane. The lipid membrane thus obtained was suspended in 8 ml of an N-tris(hydroxymethyl)methyl-3-aminopropanesulfonate buffer-physiological saline solution (TAPS, pH 8.4) and sonicated, giving 8 ml of a clear micelle suspension. Docetaxel hydrate that was completely dissolved in PBS buffer (pH 7.2) to a concentration of 20 mg/2 ml was slowly added dropwise under stirring to the micelle suspension and uniformly mixed, following which the resulting docetaxel hydrate-containing micelle suspension was subjected to ultrafiltration using a PM10 membrane (Amicon Co., USA) and PBS buffer-physiological saline (pH 7.2), thereby giving 10 ml of a uniform docetaxel hydrate-encapsulated liposome particle suspension (abbreviated below as "DC-1 suspension").

### (2) Hydrophilizing Treatment of Lipid Membrane Surface of Anticancer Drug Docetaxel-Encapsulated Liposomes

Ten milliliters of the DC-1 suspension was subjected to ultrafiltration using an XM300 membrane (Amicon Co., USA) and sodium bicarbonate buffer-physiological saline (CBS, pH 8.5), thereby setting the pH of the solution to 8.5. Next, 10 mg of the crosslinking reagent bis(sulfosuccinimidyl) suberate (BS3; Pierce Co., USA) was added, and stirring was carried out at 25°C for 2 hours. The mixture was additionally stirred overnight at 7°C, thereby bringing to completion the chemical binding reaction between the lipid dipalmitoylphosphatidylethanolamine on the liposome membranes and BS3. This liposome solution was then subjected to ultrafiltration using an XM300 membrane and a CBS buffer (pH 8.5). Next, 40 mg of tris(hydroxymethyl)aminomethane dissolved in 1 ml of CBS buffer (pH 8.5) was added to 10 ml of the liposome solution, after which the mixture was stirred for 2 hours at 25°C, then overnight at 7°C, thereby bringing the chemical binding reaction between the BS3 bonded to the lipid on the liposome membrane and tris(hydroxymethyl)aminomethane to completion. The hydroxyl groups of tris(hydroxymethyl)aminomethane were attached in this way to the lipid dipalmitoylphosphatidylethanolamine in the membrane of the docetaxel hydrate-encapsulated liposome, resulting in hydrophilization by hydration. This product is referred to below as "DC-2 suspension."

### (3) Coupling Human Serum Albumin (HSA) to Membrane Surface of Docetaxel Hydrate-Encapsulated Liposomes

Human serum albumin (HSA) was bound to the DC-2 suspension by employing a coupling reaction in accordance with the method described in Yamazaki, N., Kodama, M and Gabius, H.J. (1994): Methods Enzymol. 242, 56-65. This reaction was carried out as a two-stage chemical reaction. First, the ganglioside present on the membrane surface of the liposomes in 10 ml of the DC-2 suspension was periodic acid oxidized by adding 43 mg of sodium metaperiodate dissolved in 1 ml of TAPS buffer (pH 8.4) and stirring the mixture at room temperature for 2 hours, following which ultrafiltration was carried out using an XM300 membrane and PBS buffer (pH 8.0), yielding 10 ml of oxidized liposomes. Twenty milligrams of human serum albumin (HSA) was added to this liposome solution and the mixture was stirred at 25°C for 2 hours, following which 100 µl of 2M NaBH₃CN in PBS (pH 8.0) was added and the mixture was stirred overnight at 10°C, thereby coupling HSA by a coupling reaction between the gangliosides on the liposomes and the HSA. Next, ultrafiltration was carried out with an XM300 membrane and a CBS buffer (pH 8.5), giving 10 ml of a HSA-coupled docetaxel hydrate-encapsulating liposome suspension (abbreviated below as "DC-3 suspension").

### (4) Attaching α-1,6-Mannobiose Disaccharide to Human Serum Albumin (HSA) Coupled to Docetaxel Hydrate-Encapsulating Liposome Membrane Surface, and Hydrophilization of Linker Protein (HSA)

After adding 150 µg of α-1,6-mannobiose disaccharide (Calbiochem Co., USA) to 0.5 ml of an aqueous solution dissolved with 0.25 g of NH₄HCO₃ and stirring at 37°C for three days, the mixture was filtered with a 0.45 µm filter and the amination reaction at the reducing ends of the sugar chains was brought to completion, giving 150 µg of the glycosylamine compound of α-1,6-mannobiose disaccharide. Next, 10 mg of the crosslinking reagent 3,3'-dithiobis(sulfosuccinimidyl) propionate (DTSSP; Pierce Co., USA) was added to 10 ml of the DC-3 suspension, following which the mixture was stirred for two hours at 25°C, then stirred overnight at 7°C and subjected to ultrafiltration with an XM300 membrane and CBS buffer (pH 8.5), giving 10 ml of liposomes in which DTSSP is bonded to the HSA on the liposomes. Next, 150 µg of the above-mentioned glycosylamine compound of α-1,6-mannobiose disaccharide was added to this liposome solution, the mixture was stirred for two hours at 25°C, and 120 mg of tris(hydroxymethyl)aminomethane (Wako Co., Japan) was added. The resulting mixture was subsequently stirred for 2 hours at 25°C, then stirred overnight at 7°C and subjected to ultrafiltration with an XM300 membrane and HEPES buffer (pH 7.2), thereby attaching the α-1,6-mannobiose disaccharide to the DTSSP on the human serum albumin coupled to the membrane surfaces of the liposomes and carrying out hydrophilizing treatment. This procedure yielded 10 ml (total lipid, 20 mg; total protein, 2 mg) of hydrophilized docetaxel hydrate-encapsulating liposomes (abbreviated below as "DC-A6";) of linker protein which were composed of α-1,6-mannobiose disaccharide, human serum albumin and liposome coupled together.

### (5) Hydrophilization of Linker Protein (HSA) by Attaching Tris(hydroxymethyl)aminomethane to Human Serum Albumin (HSA) Coupled to docetaxel hydrate-encapsulating liposome Membrane Surface

Ten milligrams of the crosslinking reagent 3,3'-dithiobis(sulfosuccinimidyl) propionate (DTSSP; Pierce Co., USA) was added to 10 ml of the DC-3 suspension, following which the mixture was stirred for two hours at 25°C, then stirred overnight at 7°C, and subjected to ultrafiltration with an XM300 membrane and CBS buffer (pH 8.5), giving 10 ml of liposomes in which DTSSP is attached to the HSA on the liposomes. Next, 120 mg of tris(hydroxymethyl)aminomethane (Wako Co., Japan) was added to this liposome solution, the mixture was stirred for two hours at 25°C then stirred overnight at 7°C, and subjected to ultrafiltration with an XM300 membrane and PBS buffer (pH 7.2), thereby carrying out attachment of the tris(hydroxymethyl)aminomethane to the DTSSP on the human serum albumin coupled to the liposome membrane surfaces. This yielded 10 ml (total lipid, 20 mg; total protein, 2 mg) of anticancer drug docetaxel-encapsulating liposomes (abbreviation: DC-TRIS;) in which the linker protein (HSA) in which the tris(hydroxymethyl)aminomethane, the human serum albumin and liposome are coupled together, was hydrophilized.

### Experiment Example 1

### (1) Quantitative Determination of Compound

The amounts of docetaxel hydrate present in DC-A6 and DC-TRIS obtained in Example 1, and the amounts of docetaxel hydrate present in animal tissue following the administration of DC-A6 and DC-TRIS were measured as described below in accordance with the method described in Anal. Chem. 77, 4677-4683 (2005).

An LC-10ADvp system (Shimadzu Corp.) was used as the HPLC system, and an API 4000 (AB/MDS SCIEX) was used as the MS/MS system.

Samples obtained by adding 5 µl of a docetaxel hydrate dilution standard and 5 µL of an internal standard solution to 50 µL of plasma from an untreated group of animals (control) were treated in the same way and used as samples for creating a working curve.

Five microliters of 0.2% formic acid-containing methanol, 5 µL of a 0.2% formic acid-containing methanol solution of paclitaxel (internal standard solution, I.S.) and 0.15 mL of acetonitrile were added to 50 µL of DC-A6, DC-TRIS, plasma samples, tissue extracts or samples for creating a standard working curve. These ingredients were stirred together, then centrifuged for 10 minutes at 4°C and 3000 rpm. The supernatant (100 µL) was collected, 0.1 mL of an HPLC mobile phase (A) was added, and the mixture was centrifuged for 10 minutes at 4°C and 3000 rpm. The sample solution thus obtained was furnished to LC/MS/MS analysis.

The samples for preparing standard working curves were prepared to the following final concentrations: 5,000 µg/mL, 2,000 µg/mL, 1,000 µg/mL, 500 µg/mL, 100 µg/mL, 50 µg/mL and 20 µg/mL.

Chromatographic separation by HPLC was carried out in gradient mode using a CAPCELL PAK C 18 MGII (particle size, 5 µg; inside diameter, 2.0 mm; length, 20 mm; Shiseido) as the separatory column. A 10 mmol/L ammonium formate/formic acid (500:1, v/v) solution was used as mobile phase (A), and methanol/formic acid (500:1, v/v) was used as mobile phase (B). Fluid delivery was carried out at a flow rate of 0.2 mL/min. The gradient program involved increasing the mobile phase (B) concentration 50% from minute 0 (at the start of chromatography) to minute 1, then linearly increasing the concentration thereafter to 90% by minute 3. Fluid delivery was subsequently carried out at a concentration of 90% up to minute 5 and the concentration was lowered to 50% after 5.1 minutes, subsequent to which fluid delivery was carried out under the same conditions up to the chromatography completion time (8 minutes). Also, using a switching valve, the eluate from minute 3.0 to minute 6 of chromatography was introduced to MS/MS. Chromatography was carried out at a column temperature of 40°C and an injection volume of 15 µL.

MS/MS analysis was performed using a turbo ion spray as the ionization mode, and the ions were detected by selected reaction monitoring (SRM) in the cationic mode. Zero air was used in the ion spray, and this step was carried out at a voltage of 4.2 kV. Nitrogen was used for collision-induced dissociation of the ions. The monitored ions, i.e., the precursor ion and the fragment ion, selected for investigation were 808.4 Da → 526.9 Da in the case of docetaxel, and 854.5 Da → 569.4 Da in the case of paclitaxel (I.S.: internal standard).

Using the peak surface area ratio between docetaxel and paclitaxel (as the internal standard (IS)), the concentration of docetaxel hydrate in each sample was computed from a recursion formula (1/concentration weighted) for the working curve.

### (2) Animal Experiments and Preparation of Specimens

The tissue (including tumor) pharmacokinetics of the docetaxel hydrate present in DC-A6 and DC-TRIS were measured using a human cancer cell nude mouse implant model. Five million human breast cancer cells BT-474 (procured from ATCC) were suspended in a Matrigel solution and transplanted subcutaneously in the chests of BALB/c female nude mice (6-week-old) (Proc. NAS 87, 6698-6720, 1990). Immediately after transplantation and on day 7 following transplantation, estradiol dipropionate (5 mg/mL solution), 50 µL, was administered intramuscularly to a hind leg for the purpose of increasing the tumor implantation rate. The tumor diameter was measured up until day 21 to 28 following transplantation, and groups of three mice having similar tumor sizes were used in the experiment.

The DC-A6 obtained in Example 1, the DC-TRIS obtained in Example 1, physiological saline (solvent control), and preparation controls (Taxotere Injection (trade name) and docetaxel hydrate injection were procured from Sanofi Aventis, and prepared in accordance with the product inserts) were each administered to the mice in doses of 0.5 mg/kg via the caudal vein. Following administration, the mice were sacrificed after 0.5, 1, 2, 4, 8 and 24 hours, and the blood, liver, kidneys, muscle and tumors were collected. The blood was promptly centrifuged (4°C, 10,000 xg) following collection, and the plasma component separated off. This was used as plasma samples. The other organs were promptly added to four-fold amounts (weight ratio) of physiological saline and rendered into complete homogenates by 3 minutes of Polytron treatment at 4°C. These homogenates were then prepared as tissue extracts and furnished for measurement of the amount of docetaxel hydrate.

### (3) Results

The docetaxel hydrate contents of DC-A6 and DC-TRIS were quantitatively determined. As a result, the docetaxel hydrate contained in DC-A6 and DC-TRIS was 63 µg/mL in each case.

Next, the docetaxel hydrate contents of tumor-containing tissues of animals given DC-A6 or DC-TRIS were measured. Docetaxel hydrate was detected from tumor samples starting 0.5 hour after administration, both in the groups given DC-A6 and the groups given DC-TRIS, demonstrating that docetaxel hydrate moves rapidly from the blood to the tumor.

As is apparent from the above, the liposomes of the invention can be used as cancer-treating preparations having excellent targetability.

### INDUSTRIAL APPLICABILITY

Sugar chain-modified liposomes having a sugar chain attached to a liposome membrane which include at least one selected from among aromatase inhibitors, anti-androgenic agents, lyase inhibitors, GnRH agonists, GnRH antagonists, anti-angiogenic agents, tyrosine kinase inhibitors, serine-threonine kinase inhibitors, antibodies having anticancer activities, ansamitocin, capecitabine, celmoleukin, docetaxel hydrate, gemcitabine hydrochloride, oxaliplatin, prednisolone, tegafur-uracil mixtures, zinostatin stimalamer and arsenic trioxide, by virtue of the structure of the sugar chains used and the amount of sugar chains attached, have differing binding activities to lectins or lectin-like proteins with sugar chain-binding sites that are present at the surface of cells in various target tissues or organs. Hence, such liposomes have an excellent targetability to tissues or organs such as blood, liver, spleen, lungs, brain, small intestine, heart, thymus, kidneys, pancreas, muscle, large intestine, bones, bone marrow, eyes, ovaries, placenta, prostate, pituitary gland, mammary glands, blood vessels, skin, gall bladder, bile ducts, bladder, adipose tissue, cancer tissue, inflamed tissue and lymph nodes, enabling the liposomes to be used as anticancer agents capable of efficiently delivering the aforementioned drugs to a target tissue or organ.

Moreover, the above sugar chain-modified liposomes of the invention have differing intestinal absorptions depending on the structure of the sugar chains used and the amount of sugar chains attached. Hence, by suitably selecting the amount of sugar chains attached to the liposomes for each type of sugar chain, the liposomes may be used as a preparation which distributes the above drugs to biological tissue by an intestinal route.

In addition, even when sugar chains are not attached to liposomes which contain at least one selected from among aromatase inhibitors, anti-androgenic agents, lyase inhibitors, GnRH agonists, GnRH antagonists, anti-angiogenic agents, tyrosine kinase inhibitors, serine-threonine kinase inhibitors, antibodies having anticancer activities, ansamitocin, capecitabine, celmoleukin, docetaxel hydrate, gemcitabine hydrochloride, oxaliplatin, prednisolone, tegafur-uracil mixtures, zinostatin stimalamer and arsenic trioxide, because the liposomes are very stable in the body, they are able to effectively deliver such drugs to various tissues or organs, such as blood, liver, spleen, lungs, brain, small intestine, heart, thymus, kidneys, pancreas, muscle, large intestine, bones, bone marrow, eyes, ovaries, placenta, prostate, pituitary gland, mammary glands, blood vessels, skin, gall bladder, bile ducts, bladder, adipose tissue, cancer tissue, inflamed tissue and lymph nodes. Accordingly, the liposomes of the invention may be utilized as anticancer agents capable of effectively delivering the above-mentioned drugs to the above-mentioned tissues or organs.

## Claims

1. A sugar chain-modified liposome comprising a sugar chain attached to a liposome membrane, the liposome containing at least one selected from among aromatase inhibitors, anti-androgenic agents, lyase inhibitors, GnRH agonists, GnRH antagonists, anti-angiogenic agents, tyrosine kinase inhibitors, serine-threonine kinase inhibitors, antibodies having anticancer activities, ansamitocin, capecitabine, celmoleukin, docetaxel hydrate, gemcitabine hydrochloride, oxaliplatin, prednisolone, tegafur-uracil mixtures, zinostatin stimalamer and arsenic trioxide.

2. The sugar chain-modified liposome according to claim 1, wherein constituent lipids in the liposome include phosphatidylcholines (molar ratio, 0 to 70%); phosphatidylethanolamines (molar ratio, 0 to 30%); one or more lipid selected from the group consisting of phosphatidic acids, long-chain alkyl phosphates and dicetyl phosphates (molar ratio, 0 to 30%); one or more lipid selected from the group consisting of gangliosides, glycolipids, phosphatidylglycerols and sphingomyelins (molar ratio, 0 to 40%); and cholesterols (molar ratio, 0 to 70%).

3. The sugar chain-modified liposome according to claim 2, wherein at least one type of lipid selected from the group consisting of gangliosides, glycolipids, phosphatidylglycerols, sphingomyelins and cholesterols collect on a surface of the liposome to form a raft.

4. The sugar chain-modified liposome according to any one of claims 1 to 3, wherein the sugar chains are attached under control of the type and density thereof.

5. The sugar chain-modified liposome according to any one of claims 1 to 4, wherein the liposome has a particle size of from 30 to 500 nm.

6. The sugar chain-modified liposome according to claim 5, wherein the liposome has a particle size of from 50 to 350 nm.

7. The sugar chain-modified liposome according to any one of claims 1 to 6, wherein the liposome has a zeta potential of from -50 to 10 mV.

8. The sugar chain-modified liposome according to claim 7, wherein the liposome has a zeta potential of from -40 to 0 mV.

9. The sugar chain-modified liposome according to claim 8, wherein the liposome has a zeta potential of from -30 to -10 mV.

10. The sugar chain-modified liposome according to any one of claims 1 to 9, wherein the sugar chain is attached to the liposome membrane through a linker protein.

11. The sugar chain-modified liposome according to claim 10, wherein the linker protein is a protein of biological origin.

12. The sugar chain-modified liposome according to claim 11, wherein the linker protein is a protein of human origin.

13. The sugar chain-modified liposome according to claim 12, wherein the linker protein is a serum protein of human origin.

14. The sugar chain-modified liposome according to claim 11, wherein the linker protein is human serum albumin or bovine serum albumin.

15. The sugar chain-modified liposome according to any one of claims 1 to 14, wherein the linker protein is coupled to a raft, which is composed of at least one type of lipid selected from the group consisting of gangliosides, glycolipids, phosphatidylglycerols, sphingomyelins and cholesterols, and which is formed on a surface of the liposome.

16. The sugar chain-modified liposome according to any one of claims 1 to 15, which is hydrophilized by attaching a hydrophilic compound to the liposome membrane and/or a linker protein.

17. The sugar chain-modified liposome according to claim 16, wherein the hydrophilic compound is a low-molecular-weight compound.

18. The sugar chain-modified liposome according to claim 16 or 17, wherein the hydrophilic compound substantially does not sterically hinder the sugar chain and does not interfere with progress of a sugar chain molecule recognition reaction by lectin on a target cell membrane surface.

19. The sugar chain-modified liposome according to any one of claims 16 to 18, wherein the hydrophilic compound has a hydroxyl group.

20. The sugar chain-modified liposome according to any one of claims 16 to 19, wherein the hydrophilic compound is an amino alcohol.

21. The sugar chain-modified liposome according to any one of claims 16 to 20, wherein the hydrophilic compound is directly attached to a surface of the liposome membrane.

22. The sugar chain-modified liposome according to claim 16, which is hydrophilized by a hydrophilic compound, the hydrophilic compound being represented by general formula (1):
X-R¹(R²OH)ₙ (1)
wherein R¹ is a C₁ to C₄₀ linear or branched hydrocarbon chain, R² is either absent or a C₁ to C₄₀ linear or branched hydrocarbon chain, X is a reactive functional group which bonds directly to a liposome lipid or a linker protein or bonds to a divalent reagent for crosslinking, and n is a positive integer.

23. The sugar chain-modified liposome according to claim 16, which is hydrophilized by a hydrophilic compound, the hydrophilic compound being represented by general formula (2):
H₂N-R³-(R⁴OH)ₙ (2)
wherein R³ is a C₁ to C₄₀ linear or branched hydrocarbon chain, R⁴ is either absent or a C₁ to C₄₀ linear or branched hydrocarbon chain, and n is a positive integer.

24. The sugar chain-modified liposome according to claim 16, which is hydrophilized by a hydrophilic compound, the hydrophilic compound being represented by the general formula (3):
H₂N-R⁵(OH)ₙ (3)
wherein R⁵ is a C₁ to C₄₀ linear or branched hydrocarbon chain, and n is a positive integer.

25. The sugar chain-modified liposome according to claim 16, wherein the liposome membrane and/or linker protein are hydrophilized by covalently bonding the hydrophilic compound, as a tris(hydroxyalkyl)aminoalkane, to the liposome membrane and/or the linker protein.

26. The sugar-chain-modified liposome according to claim 25, wherein the liposome membrane and/or linker protein are hydrophilized by covalently bonding a hydrophilic compound selected from the group consisting of tris(hydroxymethyl)aminoethane, tris(hydroxyethyl)aminoethane, tris(hydroxypropyl)aminoethane, tris(hydroxymethyl)aminomethane, tris(hydroxyethyl)aminomethane, tris(hydroxypropyl)aminomethane, tris(hydroxymethyl)aminopropane, tris(hydroxyethyl)aminopropane and tris(hydroxypropyl)aminopropane to the liposome membrane and/or the linker protein.

27. The sugar chain-modified liposome according to any one of claims 1 to 26, wherein the sugar chain-modified liposome targets, as a receptor present on cell membrane surfaces of various tissues, a lectin selected from the group consisting of C-type lectins including selectin, DC-SIGN, DC-SGNR, collectin and mannose-binding lectins, I-type lectins including Siglec, P-type lectins including mannose-6-phosphate receptors, R-type lectins, L-type lectins, M-type lectins and galectin.

28. The sugar chain-modified liposome according to claim 27, wherein the sugar chain-modified liposome targets a selectin selected from the group consisting of E-selectin, P-selectin and L-selectin.

29. The sugar chain-modified liposome according to any one of claims 1 to 28, which has a sugar chain bonding density per liposome particle of from 1 to 30,000 sugar chains when a linker protein is used, and of from 1 to 500,000 sugar chains when a linker protein is not used.

30. The sugar chain-modified liposome according to any one of claims 1 to 28, which includes a linker protein and has a sugar chain bonding density per linker protein molecule of from 1 to 60 sugar chains.

31. The sugar chain-modified liposome according to any one of claims 1 to 30, wherein the liposome has an intestinal absorbability.

32. The sugar chain-modified liposome according to any one of claims 1 to 31, which has a high targetability to a tissue or organ selected from the group consisting of blood, blood cells, liver, spleen, lungs, brain, small intestine, heart, thymus, kidneys, pancreas, muscle, large intestine, bones, bone marrow, eyes, ovaries, placenta, prostate, pituitary gland, mammary glands, blood vessels, skin, gall bladder, bile ducts, bladder, adipose tissue, cancer tissue, inflamed tissue and lymph nodes.

33. The sugar chain-modified liposome according to any one of claims 1 to 32, wherein the sugar chain is selected from the group consisting of α-1,2-mannobiose disaccharide, α-1,3-mannobiose disaccharide, α-1,4-mannobiose disaccharide, α-1,6-mannobiose disaccharide, α-1,3-α-1,6-mannotriose trisaccharide, oligomannose-3 pentasaccharide, oligomannose-4b hexasaccharide, oligomannose-5 heptasaccharide, oligomannose-6 octasaccharide, oligomannose-7 nonasaccharide, oligomannose-8 decasaccharide, oligomannose-9 undecasaccharide, 3'-sialyllactose trisaccharide, 6'-sialyllactose trisaccharide, 3'-sialyllactosamine trisaccharide, 6'-sialyllactosamine trisaccharide, Lewis X trisaccharide, sialyl Lewis X tetrasaccharide, lactose disaccharide, 2'-fucosyllactose trisaccharide, difucosyllactose tetrasaccharide and 3-fucosyllactose trisaccharide.

34. A liposomal preparation comprising the liposome according to any one of claims 1 to 33.

35. The liposomal preparation according to claim 34, which is an oral preparation.

36. The liposomal preparation according to claim 34, which is a parenteral preparation.

37. A liposome comprising a membrane which is hydrophilized and to a surface of which sugar chains are not attached, the liposome containing at least one selected from among aromatase inhibitors, anti-androgenic agents, lyase inhibitors, GnRH agonists, GnRH antagonists, anti-angiogenic agents, tyrosine kinase inhibitors, serine-threonine kinase inhibitors, antibodies having anticancer activities, ansamitocin, capecitabine, celmoleukin, docetaxel hydrate, gemcitabine hydrochloride, oxaliplatin, prednisolone, tegafur-uracil mixtures, zinostatin stimalamer and arsenic trioxide.

38. The liposome according to claim 37, wherein constituent lipids in the liposome include phosphatidylcholines (molar ratio, 0 to 70%); phosphatidylethanolamines (molar ratio, 0 to 30%); one or more lipid selected from the group consisting of phosphatidic acids, long-chain alkyl phosphates and dicetyl phosphates (molar ratio, 0 to 30%); one or more lipid selected from the group consisting of gangliosides, glycolipids, phosphatidylglycerols and sphingomyelins (molar ratio, 0 to 40%); and cholesterols (molar ratio, 0 to 70%).

39. The liposome according to claim 37 or 38, which further includes a protein.

40. The liposome according to any one of claims 37 to 39, which is hydrophilized by attaching a hydrophilic compound to the liposome membrane and/or a linker protein.

41. The liposome according to claim 40, wherein the hydrophilic compound is a low-molecular-weight substance.

42. The liposome according to claim 40 or 41, wherein the hydrophilic compound substantially does not sterically hinder the sugar chain and does not interfere with progress of a sugar chain molecule recognition reaction by lectin on a target cell membrane surface.

43. The liposome according to any one of claims 40 to 42, wherein the hydrophilic compound has a hydroxyl group.

44. The liposome according to any one of claims 40 to 43, wherein the hydrophilic compound is an amino alcohol.

45. The liposome according to any one of claims 40 to 44, wherein the hydrophilic compound is directly attached to a surface of the liposome membrane.

46. The liposome according to claim 40, wherein the low-molecular-weight hydrophilic compound has at least one hydroxyl group.

47. The liposome according to claim 40, wherein the hydrophilic compound is represented by general formula (1):
X-R¹(R²OH)ₙ (1)
wherein R¹ is a C₁ to C₄₀ linear or branched hydrocarbon chain, R² is either absent or a C₁ to C₄₀ linear or branched hydrocarbon chain, X is a reactive functional group which bonds either directly to a liposome lipid or a linker protein or bonds to a crosslinking divalent reagent, and n is a positive integer.

48. The liposome according to claim 40, wherein the hydrophilic compound is represented by general formula (2):
H₂N-R³-(R⁴OH)ₙ (2)
wherein R³ is a C₁ to C₄₀ linear or branched hydrocarbon chain, R⁴ is either absent or a C₁ to C₄₀ linear or branched hydrocarbon chain, and n is a positive integer.

49. The liposome according to claim 40, wherein the hydrophilic compound is represented by general formula (3):
H₂N-R⁵(OH)ₙ (3)
wherein R⁵ is a C₁ to C₄₀ linear or branched hydrocarbon chain, and n is a positive integer.

50. The liposome according to claim 40, wherein the liposome membrane and/or linker protein are hydrophilized by covalently bonding the hydrophilic compound as a tris(hydroxyalkyl)aminoalkane to the liposome membrane and/or the linker protein.

51. The liposome according to claim 50, wherein the liposome membrane and/or linker protein are hydrophilized by covalently bonding a hydrophilic compound selected from the group consisting of tris(hydroxymethyl)aminoethane, tris(hydroxyethyl)aminoethane, tris(hydroxypropyl)aminoethane, tris(hydroxymethyl)aminomethane, tris(hydroxyethyl)aminomethane, tris(hydroxypropyl)aminomethane, tris(hydroxymethyl)aminopropane, tris(hydroxyethyl)aminopropane and tris(hydroxypropyl)aminopropane to the liposome membrane and/or the linker protein.

52. A liposomal preparation comprising the liposome according to any one of claims 37 to 51.

53. A liposomal preparation comprising the liposome according to claim 52, which is an oral preparation.

54. A liposomal preparation comprising the liposome according to claim 52, which is a parenteral preparation.
